(19) Europäisches Patentamt
European Patent Office
Office européen des brevets

(11) EP 1 447 411 A1

(12) EUROPEAN PATENT APPLICATION
published in accordance with Art. 158(3) EPC

(43) Date of publication:
**18.08.2004 Bulletin 2004/34**

(21) Application number: **02778030.3**

(22) Date of filing: **01.11.2002**

(51) Int Cl.[7]: **C07K 14/47**, C12N 15/12, C12N 5/10, C12N 1/15, C12N 1/19, C12N 1/21, C12P 21/02, C07K 16/18, C12Q 1/02, A61K 38/16, A61K 39/395, A61K 45/00, A61P 11/00

(86) International application number:
**PCT/JP2002/011417**

(87) International publication number:
**WO 2003/037927 (08.05.2003 Gazette 2003/19)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **02.11.2001 JP 2001337864**
**13.12.2001 JP 2001380099**
**18.01.2002 JP 2002010035**

(71) Applicant: **Takeda Chemical Industries, Ltd.**
**Osaka-shi, Osaka 541-0045 (JP)**

(72) Inventors:
- **NAKANISHI, Atsushi**
  **Tsukuba-shi, Ibaraki 305-0025 (JP)**
- **MORITA, Shigeru**
  **Tsukuba-shi, Ibaraki 300-3261 (JP)**

(74) Representative: **Rickard, Timothy Mark Adrian**
**Takeda Patent Office,**
**11-12 Charles II Street**
**London SW1Y 4QU (GB)**

# (54) NOVEL PROTEIN, ITS DNA AND USE THEREOF

(57) The present invention provides a novel protein which is useful in preventing/treating pulmonary and breast diseases associated with pulmonary and airway inflammation, a polynucleotide encoding the protein, a method of screening a compound inhibiting the activity of the protein, a compound obtained by the screening method, etc.

The protein and polynucleotide of the present invention are useful as diagnostic markers for pulmonary and breast disease associated with pulmonary and airway inflammation, rhinitis and the like. Inhibitors obtained by screening with the use of the protein or the polynucleotide and a neutralizing antibody inhibiting the activity of the protein are usable as agents for the prevention/treatment of, for example, pulmonary and breast diseases associated with pulmonary and airway inflammation such as chronic obstructive pulmonary disease, rhinitis and the like.

EP 1 447 411 A1

## Description

FIELD OF THE INVENTION

The present invention relates to a novel protein, a DNA encoding the protein, a method of screening a compound inhibiting the activity of the protein, a compound obtained by the screening method, etc. More particularly, the present invention relates to a novel protein, which is useful as an agent for the prevention/treatment of or a diagnostic agent of chronic obstructive pulmonary disease.

BACKGROUND ART

Chronic obstructive pulmonary disease (chronic bronchitis, pulmonary emphysema, diffuse panbronchiolitis, intrinsic asthma, etc.) is considered to be a main disease in respiratory diseases in the future, as the smoking generation is aging, life expectancy has increased, etc.
Also, bronchial asthma is a chronic inflammatory disease of the airways showing respiratory stenosis, which symptom observed includes paroxysmal dyspnea, stridor, cough, etc. Many cells such as respiratory epithelial cells, mast cells, eosinophils, T lymphocytes, etc. take part in its onset and development. One of the most important characteristics of bronchial asthma is that the airways are hyperresponsive to irritants (airway hyperresponsiveness). This airway hyperresponsiveness is attributable to respiratory inflammation caused chiefly due to desquamation of respiratory epithelial cells by neurotransmitters secreted from the cells such as eosinophils, etc., infiltrated into the airways, and it is also considered that genetic factors or environmental factors will affect the airway hyperresponsiveness complicatedly.
When the inflammatory reaction of the airways is triggered by external irritants (allergens, exhausts) or viral infection, adhesion molecules including VCAM-1, ICAM-1 and the like are expressed on respiratory epithelial cells or on capillary endothelial cells around the bronchi [J. Allergy Clin. Immunol., 96, 941 (1995)] to produce cytokines or chemotactic substances. In patients with bronchial asthma, the function of Th2 type helper T cells is accentuated to increase the production of Th2 type cytokines such as IL-3, IL-4, IL-5, IL-13, GM-CSF, etc., or chemokines such as eotaxin, RANTES, etc. IL-4 or IL-13 has an activity of inducing IgE production, and IL-3 or IL-4 has an activity of inducing the growth of mast cells. Furthermore, eosinophils are differentiated and proliferated by the action of IL-5, GM-CSF, etc. and mediated by eotaxin or RANTES to infiltrate into the airways [Allergy Asthma Proc., 20, 141 (1999)].
In particular, among the cytokines IL-13 is reportedly an important factor for the development of chronic obstructive pulmonary disease or mucus hypersecretion in bronchial asthma [J. Clin. Invest., 106, 1081 (2000), J. Clin. Invest., 103, 779 (1999)].
The base sequence of human CLCA1 gene is described in Genomics, 54, 200 (1998), Biochem. Biophys. Res. Commun., 255, 347 (1999), etc.; it is reported that this gene is associated with bronchial asthma and chronic obstructive pulmonary disease (Patent Article 1: WO 01/38530).
The base sequences of Mouse gob-5 [Biochem. Biophys. Res. Commun., 255, 347 (1999)], porcine CLCA1 gene [Physiol. Genomics, 3, 101 (2000)], etc. are known to show homology to the base sequence of human CLCA1 gene.

DISCLOSURE OF THE INVENTION

It has been desired to develop an excellent agent for the prevention/treatment or diagnostic agent for chronic obstructive pulmonary disease and bronchial asthma, with minimized side effects.
The present inventors have made extensive studies to solve the foregoing problems. As a result, the inventors have found a cDNA, which expression is induced by IL-13, and come to accomplish the present invention.
That is, the present invention provides the following features:

(1) A protein containing the same or substantially the same amino acid sequence as the amino acid sequence represented by SEQ ID NO: 1, or a salt thereof;
(2) A protein consisting of the amino acid sequence represented by SEQ ID NO: 1, or a salt thereof;
(3) A protein containing the same or substantially the same amino acid sequence as the amino acid sequence represented by SEQ ID NO: 22, or a salt thereof;
(4) A protein consisting of the amino acid sequence represented by SEQ ID NO: 22, or a salt thereof;
(5) A partial peptide of the protein according to (1) or (3), or a salt thereof;
(6) A polynucleotide containing a polynucleotide encoding the protein according to (1) or (3), or the partial peptide according to (5);
(7) The polynucleotide according to (6), which is a DNA;

(8) A DNA consisting of the base sequence represented by SEQ ID NO: 2;
(9) A DNA consisting of the base sequence represented by SEQ ID NO: 23;
(10) A recombinant vector containing the polynucleotide according to (7);
(11) A transformant transformed by the recombinant vector according to (10);
(12) A method of manufacturing the protein according to (1) or (3) or the partial peptide according to (5), or a salt thereof, which comprises culturing the transformant according to (11), producing/accumulating the protein according to (1) or (3) or the partial peptide according to (5), or a salt thereof, and collecting the same;
(13) A diagnostic product comprising the polynucleotide according to (7);
(14) An antibody to the protein according to (1) or (3) or the partial peptide according to (5), or a salt thereof;
(15) A pharmaceutical comprising the antibody according to (14);
(16) A diagnostic product comprising the antibody according to (14);
(17) An antisense polynucleotide comprising a complementary or substantially complementary base sequence to that of the polynucleotide according to (7), or a part thereof;
(18) A pharmaceutical comprising the antisense polynucleotide according to (17);
(19) A method of screening a compound or its salt that inhibits the activity of the protein according to (1) or (3) or the partial peptide according to (5), or a salt thereof, which comprises using the protein according to (1) or (3) or the partial peptide according to (5), or a salt thereof;
(20) A kit for screening a compound or its salt that inhibits the activity of the protein according to (1) or (3) or the partial peptide according to (5), or a salt thereof, comprising the protein according to (1) or (3) or the partial peptide according to (5), or a salt thereof;
(21 ) A compound or its salt that inhibits the activity of the protein according to (I) or (3) or the partial peptide according to (5), or a salt thereof, which is obtainable using the screening method according to (19) or the screening kit according to (20);
(22) A pharmaceutical comprising the compound or its salt according to (21);
(23) A method of screening a compound or its salt that inhibits expression of a gene for the protein according to (1) or (3), which comprises using the polynucleotide according to (6);
(24) The screening method according to (23), wherein a cytokine is further used;
(25) The screening method according to (24), wherein the cytokine is IL-13;
(26) A kit for screening a compound or its salt that inhibits expression of a gene for the protein according to (1) or (3), which is characterized by comprising the polynucleotide according to (6);
(27) A method of screening a compound or its salt that inhibits expression of a gene for the protein according to (1) or (3), which comprises determining expression levels of a gene for the protein according to (1) or (3) or the partial peptide according to (5), when (i) a cell capable of producing the protein according to (1) or (3) or the partial peptide according to (5) is cultured in the presence of a cytokine and (ii) a mixture of the cell and a test compound is cultured in the presence of a cytokine, and comparing the expression levels;
(28) A compound or its salt that inhibits expression of a gene for the protein according to (1) or (3), which is obtainable using the screening method according to (23) or (27) or the screening kit according to (26);
(29) A pharmaceutical comprising the compound or its salt according to (28);
(30) The pharmaceutical according to (15), (18), (22) or (29), which is an agent for the prevention/treatment of chronic obstructive pulmonary disease or bronchial asthma;
(31) The diagnostic product according to (13) or (16), which is a diagnostic product of chronic obstructive pulmonary disease or bronchial asthma;
(32) A method of preventing/treating chronic obstructive pulmonary disease or bronchial asthma, which comprises administering an effective dose of the compound or its salt according to (21) or (28) to a mammal;
(33) Use of the compound or its salt according to (21) or (28) for manufacturing an agent for the prevention/treatment of chronic obstructive pulmonary disease or bronchial asthma;
(34) An agent for the prevention/treatment of pulmonary and breast diseases, comprising a compound or its salt that inhibits the activity of the protein according to (1) or (3) or the partial peptide according to (5), or a salt thereof; etc.

The present invention further provides the following features:

(35) The protein according to (1), which is a protein containing 1) the amino acid sequence represented by SEQ ID NO: 1, 2) the amino acid sequence represented by SEQ ID NO: 1, wherein at least 1 or 2 (preferably approximately 1 to 30, more preferably approximately 1 to 10 and most preferably several (1 to 5)) amino acids are deleted; 3) the amino acid sequence represented by SEQ ID NO: 1, to which at least 1 or 2 (preferably approximately 1 to 30, more preferably approximately 1 to 10 and most preferably several (1 to 5)) amino acids are added; 4) the amino acid sequence represented by SEQ ID NO: 1, in which at least 1 or 2 (preferably approximately 1 to 30, more preferably approximately 1 to 10 and most preferably several (1 to 5)) amino acids are inserted; 5) the amino acid sequence represented by SEQ ID NO: 1, in which at least 1 or 2 (preferably approximately 1 to 30, more preferably approximately 1 to 10 and most preferably several (1 to 5)) amino acids are substituted by other amino acids; or 6) a combination of these amino acid sequences 2) to 5);

(36) The protein according to (3), which is a protein containing 1) the amino acid sequence represented by SEQ ID NO: 22, 2) the amino acid sequence represented by SEQ ID NO: 22, wherein at least 1 or 2 (preferably approximately 1 to 30, more preferably approximately 1 to 10 and most preferably several (1 to 5)) amino acids are deleted; 3) the amino acid sequence represented by SEQ ID NO: 22, to which at least 1 or 2 (preferably approximately 1 to 30, more preferably approximately 1 to 10 and most preferably several (1 to 5)) amino acids are added; 4) the amino acid sequence represented by SEQ ID NO: 22, in which at least 1 or 2 (preferably approximately 1 to 30, more preferably approximately 1 to 10 and most preferably several (1 to 5)) amino acids are inserted; 5) the amino acid sequence represented by SEQ ID NO: 22, in which at least 1 or 2 (preferably approximately 1 to 30, more preferably approximately 1 to 10 and most preferably several (1 to 5)) amino acids are substituted by other amino acids; or 6) a combination of these amino acid sequences 2) to 5);

(37) A polynucleotide, which hybridizes to the polynucleotide according to (6) under high stringent conditions; etc.

BRIEF DESCRIPTION OF THE DRAWINGS

FIG. 1 shows the induction of expression of rat CLCA1 gene in rat primary airway epithelial cells in the presence of mouse IL-13, human IL-13, mouse IL-4, human IL-4 or human IL-9 in EXAMPLE 2. In the figure, the ordinate designates a copy number of rat CLCA1 genes per GAPDH gene.

FIG. 2 shows an inhibitory action of wortmannin on the induction of expression of rat CLCA1 gene by IL-13 in SPOC-1 cells in EXAMPLE 4. In the figure, the ordinate designates luminescence intensity in the presence of wortmannin when control is made 100% luminescence intensity, wherein white, grey and black bars represent expression levels of rat CLCA1 genes, when wortmannin was added in 100 μM, 10 μM and 1 μM, respectively.

BEST MODE FOR CARRYING OUT THE INVENTION

In the present invention the protein containing the same or substantially the same amino acid sequence as the amino acid sequence represented by SEQ ID NO: 1 and the protein containing the same or substantially the same amino acid sequence as the amino acid sequence represented by SEQ ID NO: 22 (hereinafter these proteins are sometimes collectively referred to as the protein of the present invention) may be any protein derived from any cells of human and non-human warm-blooded animals (e.g., guinea pig, rat, mouse, chicken, rabbit, swine, sheep, bovine, monkey, etc.) such as hepatocyte, splenocyte, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence having substantially the same amino acid sequence as that represented by SEQ ID NO: 1 includes amino acid sequences having at least about 90% homology, preferably at least about 95% homology, more preferably at least about 97% homology and most preferably at least about 99% homology, to the amino acid sequence shown by SEQ ID NO: 1; etc.

Preferred examples of the protein containing substantially the same amino acid sequence as the amino acid sequence represented by SEQ ID NO: 1 include proteins having substantially the same amino acid sequence as the amino acid sequence represented by SEQ ID NO: 1 and having a property substantially equivalent to that of the protein having the amino acid sequence represented by SEQ ID NO: 1, etc.

The amino acid sequence having substantially the same amino acid sequence as that represented by SEQ ID NO: 22 includes amino acid sequences having at least about 90% homology, preferably at least about 95% homology, more preferably at least about 97% homology and most preferably at least about 99% homology, to the amino acid sequence shown by SEQ ID NO: 22; etc.

Preferred examples of the protein containing substantially the same amino acid sequence as the amino acid sequence represented by SEQ ID NO: 22 include proteins having substantially the same amino acid sequence as the amino acid sequence represented by SEQ ID NO: 1 and having a property substantially equivalent to that of the protein having the amino acid sequence represented by SEQ ID NO: 22, etc.

As the substantially equivalent properties, there are, for example, include a chloride channel-like activity (calcium-dependent chloride channel-like activity) and the like. The substantially equivalent is used to mean that the nature of these properties is equivalent in terms of quality (e.g., physiologically or pharmacologically). Thus, the chloride channel-like activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

These activities such as the chloride channel-like activity, etc. can be determined by publicly known methods with modifications. For example, the activities can be assayed by the method described in Genomics, 54, 200 (1998) or with modifications thereof.

Examples of the protein of the present invention include (1) so-called muteins such as proteins containing 1) the amino acid sequence represented by SEQ ID NO: 1, of which at least 1 or 2 (preferably about 1 to about 30, more preferably about 1 to about 10 and most preferably several (1 to 5)) amino acids are deleted; 2) the amino acid sequence represented by SEQ ID NO: 1, to which at least 1 or 2 (preferably about 1 to about 30, more preferably about 1 to about 10 and most preferably several (1 to 5)) amino acids are added; 3) the amino acid sequence represented by SEQ ID NO: 1, in which at least 1 or 2 (preferably about 1 to about 30, more preferably about 1 to about 10 and most preferably several (1 to 5)) amino acids are inserted; 4) the amino acid sequence represented by SEQ ID NO: 1, in which at least 1 or 2 (preferably about 1 to about 30, more preferably about 1 to about 10 and most preferably several (1 to 5)) amino acids are substituted by other amino acids; or 5) a combination of these amino acid sequences; and, (2) so-called muteins such as proteins containing 1) the amino acid sequence represented by SEQ ID NO: 22, of which at least 1 or 2 (preferably about 1 to about 30, more preferably about 1 to about 10 and most preferably several (1 to 5)) amino acids are deleted; 2) the amino acid sequence represented by SEQ ID NO: 22, to which at least 1 or 2 (preferably about 1 to about 30, more preferably about 1 to about 10 and most preferably several (1 to 5)) amino acids are added; 3) the amino acid sequence represented by SEQ ID NO: 22, in which at least 1 or 2 (preferably about 1 to about 30, more preferably about 1 to about 10 and most preferably several (1 to 5)) amino acids are inserted; 4) the amino acid sequence represented by SEQ ID NO: 22, in which at least 1 or 2 (preferably about 1 to about 30, more preferably about 1 to about 10 and most preferably several (1 to 5)) amino acids are substituted by other amino acids; or 5) a combination of these amino acid sequences; etc.

Throughout the specification, the proteins are represented in accordance with the conventional way of describing proteins, that is, the N-terminus (amino terminus) at the left hand and the C-terminus (carboxyl terminus) at the right hand. In the protein of the present invention including the protein containing the amino acid sequence shown by SEQ ID NO: 1 or SEQ ID NO: 22, the C-terminus may be in any form of a carboxyl group (-COOH), a carboxylate (-COO-), an amide ($-CONH_2$) and an ester (-COOR).

Herein, examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl such as a phenyl-$C_{1-2}$ alkyl group, e.g., benzyl, phenethyl, etc.; an $\alpha$-naphthyl-$C_{1-2}$ alkyl group such as $\alpha$-naphthylmethyl, etc.; pivaloyloxymethyl and the like.

Where the protein of the present invention contains a carboxyl group (or a carboxylate) at a position other than the C-terminus, the carboxyl group may be amidated or esterified and such an amide or ester is also included within the protein of the present invention. Examples of the ester group in this case may be the C-terminal esters described above, etc.

Furthermore, examples of the protein of the present invention include variants wherein the amino group at the N-terminal amino acid residues (e.g., methionine residue) is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group, e.g., formyl group, acetyl group, etc.), or conjugated proteins such as glycoproteins having sugar chains; etc.

Specific examples of the protein of the present invention are a protein containing the amino acid sequence represented by SEQ ID NO: 1, a protein containing the amino acid sequence represented by SEQ ID NO: 22 and the like.

The partial peptide of the protein of the present invention may be any peptide as long as it is a partial peptide of the protein of the present invention described above and preferably has the property equivalent to that of the protein of the present invention described above. Preferably used are peptides containing, e.g., at least 20, preferably at least 50, more preferably at least 70, much more preferably at least 100, and most preferably at least 200, amino acids in the constituent amino acid sequence of the protein of the present invention, and the like.

The partial peptide of the present invention may be peptides containing the amino acid sequence, of which at least 1 or 2 (preferably about 1 to about 10 and more preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 10, more preferably several and most preferably about 1 to about 5) amino acids may be substituted by other amino acids.

In the partial peptide of the present invention, the C-terminus may be in any form of a carboxyl group (-COOH), a carboxylate (-COO-), an amide ($-CONH_2$) or an ester (-COOR).

Furthermore, the partial peptide of the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as so-called glycoproteins having sugar chains; etc., as in the protein of the present invention described above.

The partial peptide of the present invention may also be used as an antigen for producing antibodies.

As salts of the protein or partial peptide of the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide of the present invention or salts thereof may be manufactured by a publicly known method used to purify a protein from human or other warm-blooded animal cells or tissues described above. Alternatively, they may also be manufactured by culturing transformants containing DNAs encoding these proteins. Furthermore, they may also be manufactured by a modification of the methods for peptide synthesis, which will be described hereinafter.

Where these proteins are manufactured from human or non-human mammalian tissues or cells, human or non-human mammalian tissues or cells are homogenized, extracted with an acid or the like, and the extract is isolated and purified by a combination of chromatography techniques such as reverse phase chromatography, ion exchange chromatography, and the like.

To synthesize the protein or partial peptide of the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which $\alpha$-amino groups and functional groups on the side chains are appropriately protected, are condensed on the resin in the order of the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobomyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower ($C_{1-6}$) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide of the present invention or salts thereof can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the partial peptide of the present invention are condensed with the remaining part. Where the product contains protecting groups, these protecting groups are removed to give the desired peptide. Publicly known methods for condensation and elimination of the protecting groups are described in i) to v) below.

i) M. Bodanszky & M.A. Ondetti: Peptide Synthesis, Interscience Publishers, New York (1966)

ii) Schroeder & Luebke: The Peptide, Academic Press, New York (1965)
iii) Nobuo Izumiya, et al.: Peptide Gosei-no-Kiso to Jikken (Basics and experiments of peptide synthesis), published by Maruzen Co. (1975)
iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the partial peptide can be converted into an appropriate salt by a publicly known method; when the partial peptide is obtained in a salt form, it can be converted into a free form or other different salt form by a publicly known method.
The polynucleotide encoding the protein of the present invention may be any polynucleotide so long as it contains the base sequence (DNA or RNA, preferably DNA) encoding the protein of the present invention described above. Such a polynucleotide may also be any one of DNA encoding the protein of the present invention, RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).
Using the polynucleotide encoding the protein of the present invention, mRNA of the protein of the present invention can be quantified by, for example, the publicly known method published in separate volume of *Jikken Igaku* 15 (7) "Shin-PCR To Sono Oyo (New PCR and its application)" (1997), or by its modifications.
The DNA encoding the protein of the present invention may be any DNA so long as it contains the base sequence encoding the protein of the present invention described above. The DNA may also be any one of genomic DNA, genomic DNA library, cDNA derived from the cells or tissues described above, cDNA library derived from the cells or tissues described above and synthetic DNA.
The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.
The DNA encoding the protein of the present invention may be, for example, (1) any one of a DNA having the base sequence represented by SEQ ID NO: 2, or any DNA having a base sequence hybridizable to a DNA having the base sequence represented by SEQ ID NO: 2 under high stringent conditions and encoding a protein which has the properties substantially equivalent to those of the protein of the present invention, (2) any one of a DNA having the base sequence represented by SEQ ID NO: 23, or any DNA having a base sequence hybridizable to a DNA having the base sequence represented by SEQ ID NO: 23 under high stringent conditions and encoding a protein which has the properties substantially equivalent to those of the protein of the present invention.
Specific examples of the DNA that is hybridizable to a DNA having the base sequence represented by SEQ ID NO: 2 under high stringent conditions include DNAs having at least about 90% homology, preferably at least about 95% homology, more preferably at least about 97% homology and most preferably at least about 99% homology, to the base sequence represented by SEQ ID NO: 2.
Specific examples of the DNA that is hybridizable to a DNA having the base sequence represented by SEQ ID NO: 23 under high stringent conditions include DNAs having at least about 90% homology, preferably at least about 95% homology, more preferably at least about 97% homology and most preferably at least about 99% homology, to the base sequence represented by SEQ ID NO: 23.
The hybridization can be carried out by publicly known methods or by a modification thereof, for example, according to the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.
The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.
More specifically, as the DNA encoding the protein having the amino acid sequence represented by SEQ ID NO: 1, there may be employed a DNA containing a DNA having the base sequence represented by SEQ ID NO: 2, etc. As the DNA encoding the protein having the amino acid sequence represented by SEQ ID NO: 22, there may be employed a DNA containing a DNA having the base sequence represented by SEQ ID NO: 23, etc. The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA.

As the DNA encoding the partial peptide of the present invention, there are employed, for example, (1) a DNA having a part of the base sequence represented by SEQ ID NO: 2, or a DNA containing a base sequence hybridizable to the base sequence represented by SEQ ID NO: 2 under high stringent conditions and containing a part of DNA encoding a protein having the activities substantially equivalent to those of the protein containing the amino acid sequence represented by SEQ ID NO: 1, (2) a DNA having a part of the base sequence represented by SEQ ID NO: 23, or a DNA containing a base sequence hybridizable to the base sequence represented by SEQ ID NO: 23 under high stringent conditions and containing a part of DNA encoding a protein having the activities substantially equivalent to those of the protein containing the amino acid sequence represented by SEQ ID NO: 22, and the like.

The DNA hybridizable to the base sequence represented by SEQ ID NO: 2 has the same significance as described above. The DNA hybridizable to the base sequence represented by SEQ ID NO: 23 has the same significance as described above.

Methods for the hybridization and the high stringent conditions that can be used are the same as those described above.

For cloning of DNAs that completely encode the protein or partial peptide of the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be screened by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or its modification, using a publicly known kit available as Mutan™-super Express Km or Mutan™-K (both manufactured by Takara Shuzo Co., Ltd.), etc.

The cloned DNA encoding the protein of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, $\lambda P_L$ promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R$^-$, NA87-11A, DKD-5D, 20B-12, Schizosaccharomyces pombe NCYC 1913, NCYC2036, Pichia pastoris KM71, etc.

Examples of insect cells include, for the virus AcNPV, Spodoptera frugiperda cell (Sf cell), MG 1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr$^-$) cell), mouse L cell, mouse AtT-20, mouse myeloma cell, rat GH 3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein of the present invention can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transfonnant is cultivated at about 27°C for about 3 days to about 5 days and, if necessary, the culture can be aerated or agitated.
Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.
As described above, the protein of the present invention can be produced in the transformant, in the cell membrane of the transformant, or outside of the transformant.
The protein of the present invention can be separated and purified from the culture described above by the following procedures.
When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.
The protein of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.
When the protein of the present invention thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.
The protein of the present invention produced by the recombinant can be treated, prior to or after the purification, with an appropriate protein-modifying enzyme so that the protein can be appropriately modified to partially remove the polypeptide. Examples of the protein-modifying enzyme include trypsin, chymotrypsin, arginyl endopeptidase, protein kinase, glycosidase and the like.
The presence of the thus produced protein of the present invention can be determined by an enzyme immunoassay or western blotting using a specific antibody.
The antibodies to the protein or partial peptide of the present invention, or its salts may be any of polyclonal and monoclonal antibodies, as long as they are capable of recognizing the protein or partial peptide of the present invention, or its salts.
The antibodies to the protein or partial peptide of the present invention, or its salts, (hereinafter they are sometimes collectively referred to as the protein of the present invention in the description of the antibodies) can be produced by a publicly known method of producing an antibody or antiserum, using the protein of the present invention as an antigen.

[Preparation of monoclonal antibody]

(a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and chickens, with the use of mice and rats being preferred.
In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

[Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantial complementary base sequence to the DNA encoding the protein or partial peptide of the present invention (hereinafter these DNAs are sometimes collectively referred to as the DNA of the present invention in the description of antisense polynucleotide) can be any antisense polynucleotide, so long as it possesses a base sequence complementary or substantially complementary base sequence to that of the DNA of the present invention and capable of suppressing expression of the DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the full-length base sequence or to the partial base sequence (i.e., complementary strand to the DNA of the present invention), and the like. Especially in the entire base sequence of the complementary strand to the DNA of the present invention, preferred is an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon).

Specific examples include an antisense polynucleotide containing the entire or part of a base sequence complementary or substantially complementary to a base sequence of DNA containing the base sequence represented by SEQ ID NO: 2 or SEQ ID NO: 23, preferably an antisense polynucleotide containing the entire or part of a base sequence complementary to a base sequence of DNA containing the base sequence represented by SEQ ID NO: 2 or SEQ ID NO: 23 (more preferably, an antisense polynucleotide containing the entire or part of a base sequence complementary to a base sequence of DNA containing the base sequence represented by SEQ ID NO: 2 or SEQ ID NO: 23), etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. These antisense nucleotides may be synthesized using a publicly known DNA synthesizer, etc.

According to the present invention, the antisense polynucleotide (nucleic acid) corresponding to the protein gene of the present invention that can inhibit replication or expression of the protein genes of the present invention can be designed and synthesized based on the base sequence information of the cloned or determined DNA. Such a polynucleotide (nucleic acid) is hybridizable to RNA of the protein gene of the present invention to inhibit the synthesis or function of said RNA or is capable of modulating or controlling the expression of the protein gene of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense" to the polynucleotides in the said target region. Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like.

Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the protein in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the DNA encoding the protein of the present invention or its partial peptides (hereinafter sometimes merely referred to as the DNA of the present invention), the antibodies to the protein of the present invention, its partial peptides, or salts thereof (hereinafter sometimes referred to as the antibodies of the present invention) and the antisense polynucleotides to the DNA of the present invention (hereinafter sometimes merely referred to as the antisense polynucleotides of the present invention) are specifically described for their applications.

The protein of the present invention can be utilized as a disease marker since expression of the protein increases tissue-specifically in chronic obstructive pulmonary disease or rhinitis. That is, the protein is useful as a marker for early diagnosis in pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like, for judgment of severity in conditions, or for predicted development of these diseases.

The pharmaceutical comprising the antisense polynucleotide of a gene encoding the protein of the present invention (the protein gene of the present invention), the compound or its salt that inhibits the activity of the protein of the present invention, expression of the protein gene, the compound or its salt that inhibits expression of the protein gene of the present invention, or the antibody to the protein of the present invention can be used as an agent for the prevention/treatment of, e.g., pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like.

[1] Screening of drug candidate compounds for disease

Since the protein of the present invention and gene for the protein of the present invention increase its expression tissue-specifically in chronic obstructive pulmonary disease or rhinitis and the protein has a mucus production accelerating activity and an activity of increasing the destruction of alveoleolar walls in the lung, airway or nasal mucosa, the compound or its salt that inhibits the activity of the protein of the present invention and the compound or its salt that inhibits expression of the gene for the protein of the present invention can be used as pharmaceuticals for the prevention/treatment of pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like.

Thus, the protein of the present invention is useful as a reagent for screening the compound or its salt that inhibits the activity of the protein of the present invention, and the polynucleotide encoding the protein is useful as a reagent for screening the compound or its salt that inhibits the protein gene of the present invention.

That is, the present invention provides (1) a method of screening the compound or its salt that inhibits the activity (e.g., a chloride channel activity, etc.) of the protein of the present invention, which comprises using the protein of the present invention (hereinafter sometimes simply referred to as the inhibitor), and (2) a method of screening the compound or its salt that inhibits expression of the protein gene of the present invention, which comprises using a polynucleotide encoding the protein of the present invention (hereinafter sometimes simply referred to as the inhibitor). In the methods of (1) and (2) described above, the screening may also be performed further by using a cytokine.

Specific examples of the screening methods include a method of screening the inhibitor, which comprises comparing (i) the case that a cell capable of producing the protein of the present invention is activated with a calcium activator and (ii) the case that a mixture of a cell capable of producing the protein of the present invention and a test compound is activated with a calcium activator; etc.

In the screening method described above, for example, the chloride channel activity of the protein of the present invention is assayed in the cases (i) and (ii), followed by comparing (i) and (ii).

The calcium activator may also be added to the cell capable of producing the protein of the present invention after mixing with a test compound.

Alternatively, the calcium activator may be added to the cell capable of producing the protein of the present invention and the test compound may then be added to the mixture.

The cell capable of producing the protein of the present invention and the test compound may be mixed at any stage before or after activation of the cell capable of producing the protein of the present invention with the calcium activator. Also, a mixture of the test compound and the calcium activator may be added to the cell capable of producing the protein of the present invention.

Ionomycin, A23187 (calcimycin), etc. are used as the calcium activators.

The chloride channel activity of the protein of the present invention can be determined by publicly known methods, e.g., the method described in Genomics, 54, 200 (1998), or its modifications.

A specific example of the screening method further includes a method of screening the inhibitor, which comprises comparing (iii) the case that a cell capable of producing the protein of the present invention is cultured in the presence of a cytokine and (iv) the case that a mixture of a cell capable of producing the protein of the present invention and a test compound is cultured in the presence of a cytokine.

Examples of cytokine are IL-13, IL-1, IL-4, IL-6, IL-8, IL-9, TNF-α, TGF-α, EGF, bFGF, etc. A preferred cytokine is IL-13.

In the screening method described above, for example, the expression level of the protein gene of the present invention (specifically, the level of the protein of the present invention or the level of mRNA encoding said protein) is determined in the cases (iii) and (iv), followed by comparing (iii) and (iv).

The cell capable of producing the protein of the present invention and the test compound may be mixed at any stage before or after the cytokine is brought in contact with the cell capable of producing the protein of the present invention. Also, a mixture of the test compound and the cytokine may be added to the cell capable of producing the protein of the present invention.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The expression level of the protein gene of the present invention can be determined by publicly known methods, e.g., in accordance with methods including Northern blotting, reverse transcription-polymerase chain reaction (RT-PCR), real time PCR monitoring system (manufactured by ABI, TaqMan polymerase chain reaction), etc., or their modifications.

Examples of the test compound include peptides, proteins, non-peptide compounds, synthetic compounds, fermentation products, cell extracts, plant extracts, animal tissue extracts, etc. These compounds may be novel compounds or publicly known compounds.

To perform the screening method described above, the cells capable of producing the protein of the present invention are suspended in a buffer suitable for the screening to prepare the cell suspension. Any buffer is usable as far as it is a buffer such as a phosphate buffer, borate buffer, etc. having pH of approximately 4 to 10 (preferably pH of about 6 to about 8) that does not interfere the chloride channel activity of the protein of the present invention.

As the cells capable of producing the protein of the present invention, there are employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the cell membrane, e.g., by culturing through the procedure described above, is preferably employed.

For example, when a test compound inhibits the chloride channel activity in the case (ii) described above by at least about 20%, preferably at least 30%, more preferably at least about 50%, as compared to the case (i) above, the test compound can be selected to be a compound capable of inhibiting the activity of the protein of the present invention, or a salt of said compound.

For example, when a test compound inhibits the expression level of the protein gene of the present invention in the case (iv) described above by at least about 20%, preferably at least 30%, more preferably at least about 50%, as compared to the case (iii) above, the test compound can be selected to be a compound capable of inhibiting the expression level of the protein gene of the present invention, or a salt of said compound.

The screening kit of the present invention comprises the protein or its partial peptide of the present invention, or a salt thereof, or the polypeptide encoding the protein or its partial peptide of the present invention, or the cell capable of producing the protein or its partial peptide of the present invention.

The compound or its salt obtained using the screening method or screening kit of the present invention is the test compound described above, the compound selected from, for example, peptides, proteins, non-peptide compounds, synthetic compounds, fermentation products, cell extracts, plant extracts, animal tissue extracts, plasma, etc., which is a compound or its salt that inhibits the activity of the protein of the present invention (e.g., the chloride channel activity, etc.) or expression of the gene for the protein of the present invention.

The salts of these compounds used are those given above as the salts of the protein of the present invention.

The compound or its salt obtained using the screening method or screening kit of the present invention is useful as pharmaceuticals for the prevention/treatment of, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like.

The compound or its salt obtained by the screening method or screening kit of the present invention may be safely administered directly as it is or in the form of an appropriate pharmaceutical. The pharmaceutical composition used for the administration described above contains a pharmacologically acceptable carrier with the aforesaid compound or its salt, a diluent or excipient. Such a composition is provided as a pharmaceutical composition suitable for oral or parenteral administration.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid antibody contained is generally 5 to 500 mg per dosage unit form; it is preferred that the aforesaid antibody is contained in about 5 to about 100 mg especially in the form of injection, and in 10 to 250 mg for the other forms.

As the composition for intranasal administration, there are used, e.g., an inhaler, nose drops, an aerosol, etc.

Where the pharmaceutical preparations obtained in a conventional manner are used as inhalers, the preparations may be used in the form of a dry powder inhaler, a suspension for inhalation, a solution for inhalation or a capsule inhaler by using publicly known methods. These preparations may be applied using an appropriate inhalator upon use, and in particular, a dry powder inhaler is preferably used.

A mean particle size of the dry powder inhaler is not particularly limited but preferably about 0.1 to about 20 μm, particularly preferably about 1 to about 5 μm.

Graininess of the dry powder inhaler is not particularly limited but preferably the particles of about 25 μm or greater are contained in about 5% or less, especially about 1% or less.

When using the inhaler, devices used for its application may be inhalators commercially available and examples include VENTOLIN ROTACAPS (Glaxo Wellcome Inc.), Spinhaler (registered trademark, Fujisawa Pharmaceutical Co., Ltd.), INTAL SPINCAPS (Fisons Ltd.), ATROVENT AND BEROTEC INHALETTEN (Boehringer Ingelheim GmbH), FORADIL (Ciba-Geigy AB), BENTODISKS (Glaxo Wellcome Inc.), PUVRIZER (registered trademark, Teijin Ltd.), BRICANYL TURBUHALER (Astra Zeneca), MIAT INSUFFLATOR, etc.

For the route of administration, these inhalers are preferably inhaled directly through nose, mouth, etc., generally using inhalators.

Since the pharmaceutical preparations thus obtained are safe and low toxic, they can be administered to human or warm-blooded animal (e.g., mouse, rat, rabbit, sheep, swine, bovine, horse, fowel, cat, dog, monkey, chimpanzee, etc.) orally or parenterally.

The dose of the compound or its salt that inhibits the activity of the protein of the present invention may vary depending upon its action, target disease, subject to be administered, route of administration, etc. For example, when the compound or its salt described above is orally administered for the treatment of, e.g., chronic obstructive pulmonary disease, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of said compound or its salt may vary depending upon subject to be administered, target disease, etc. When the compound or its salt that inhibits the activity of the protein of the present invention is administered to an adult (as 60 kg body weight) in the form of an injectable preparation for the treatment of, e.g., chronic obstructive pulmonary disease, it is advantageous to administer the compound or its salt intravenously in a daily dose of about 0.01 to about 30 mg, preferably about 0.1 to about 20 mg, and more preferably about 0.1 to about 10 mg. For other animal species, the corresponding dose as converted per 60 kg weight can be administered.

[2] Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:

(i) A method for quantification of the protein of the present invention in a test sample fluid, which comprises competitively reacting the antibody of the present invention, a test sample fluid and the labeled form of the protein of the present invention, and measuring the ratio of the labeled form of the protein of the present invention bound to said antibody; and,

(ii) A method for quantification of the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or $F(ab')_2$, Fab' or Fab fractions of the antibody molecule may also be used.

The method for quantification of the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [$^{125}$I], [$^{131}$I], [$^{3}$H], [$^{14}$C], etc. The enzymes described above are preferably enzymes, which are stable and have a high specific activity, and include, e.g., β-galactosidase, β-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antibody to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibody of the present invention.

Furthermore, when an increased level of the protein of the present invention is detected by quantifying the level of the protein of the present invention using the antibody of the present invention, it can be diagnosed that one suffers from diseases, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), etc.; or it is highly likely to suffer from these disease in the future.

Moreover, the antibody of the present invention can be used to detect the protein of the present invention, which is present in a test sample fluid such as a body fluid, a tissue, etc. The antibody can also be used to prepare an antibody column for purification of the protein of the present invention, detect the protein of the present invention in each fraction upon purification; analyze the behavior of the protein of the present invention in the cells under investigation; etc.

[3] Gene diagnostic agent

By using the DNA of the present invention, e.g., as a probe, an abnormality (gene abnormality) of the DNA or mRNA encoding the protein of the present invention in human or warm-blooded animal (e.g., rat, mouse, guinea pig, rabbit, fowel, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., the Northern hybridization or DNA mutation is detected by the PCR-SSCP assay, it can be diagnosed that it is highly likely to suffer from diseases, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), etc.

[4] Pharmaceutical comprising an antisense polynucleotide

The antisense polynucleotide of the present invention that binds to the DNA of the present invention complementarily to inhibit expression of the DNA is low toxic and can suppress the functions of the protein of the present invention or the DNA of the present invention in vivo. Thus, the antisense polynucleotide can be used as an agent for the prevention/treatment of diseases, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), etc.

Where the antisense polynucleotide is used as the agent for the prevention/treatment described above, it can be prepared into pharmaceutical preparations by publicly known methods, which are provided for administration.

For example, when the antisense polynucleotide is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or non-human mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

The dose of the antisense polynucleotide may vary depending on target disease, subject to be administered, route for administration, etc. When the antisense polynucleotide of the present invention is topically administered into the trachea in the form of an inhaler for the treatment of, e.g., chronic obstructive pulmonary disease, the antisense polynucleotide is generally administered to an adult (body weight of 60 kg) in a daily dose of about 0.1 to about 100 mg.

In addition, the antisense polynucleotide may also be used as an oligonucleotide probe for diagnosis to examine the presence of the DNA of the present invention in tissues or cells and states of its expression.

The present invention further provides:

(1) Double-stranded RNA containing a part of RNA encoding the protein of the present invention;
(2) A pharmaceutical comprising said double-stranded RNA;
(3) A ribozyme containing a part of RNA encoding the protein of the present invention; and
(4) A pharmaceutical comprising said ribozyme.

Since the double-stranded RNA (RNAi; RNA interference method), ribozyme and the like can suppress the expression of the polynucleotide (e.g., DNA) of the present invention, as the antisense polynucleotide described above can, and the in vivo function of the protein of the present invention or the polynucleotide (e.g., DNA) of the present invention, it can be used as an agent for the prevention/treatment of diseases, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes an adjacent portion to a cleavage site on the RNA of the present invention, which may be cleaved by a publicly known ribozyme (RNA fragment).

Where the double-stranded RNA or ribozyme described above can be used as the preventive/therapeutic agent described above, the ribozyme can be prepared into pharmaceutical preparations, which are provided for administration, as in the antisense polynucleotide.

[5] Pharmaceutical comprising the antibody of the present invention

The antibody having the action of neutralizing the activity of the protein of the present invention can be used as pharmaceuticals for diseases including, e.g., pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), or the like.

The agent for the prevention/treatment described above comprising the antibody of the present invention is low toxic and can be administered to human or non-human mammal (e.g., rat, rabbit, sheep, swine, bovine, cat, dog, monkey, etc.) orally or parenterally in the form of liquid as it is or as a pharmaceutical composition in a suitable preparation. The dose may vary depending upon subject to be administered, target disease, conditions, route of administration, etc. In general, when it is used for the prevention/treatment of, e.g., chronic obstructive pulmonary disease, it is advantageous to administer the antibody of the present invention intravenously to an adult in a single dose of about 0.01 to about 20 mg/kg body weight, preferably about 0.1 to about 10 mg/kg body weight, more preferably about 0.1 to about 5 mg/kg body weight, in about 1 to about 5 times per day, preferably in about 1 to about 3 times per day. In other parenteral administration and oral administration, the antibody can be administered in a dose corresponding to the above dose. When the condition is especially severe, the dose may be increased accordingly to the condition.

The antibody of the present invention may be administered in itself or as an appropriate pharmaceutical composition. The pharmaceutical composition used for the administration described above contains a pharmacologically acceptable carrier with the aforesaid antibody or its salts, a diluent or excipient. Such a composition is provided in the form of pharmaceutical preparations suitable for oral or parenteral administration.

That is, examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate dissolution aid such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a dissolution aid such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid antibody contained is generally 5 to 500 mg per dosage unit form; especially in the form of injection, it is preferred that the aforesaid antibody is contained in 5 to 100 mg and in 10 to 250 mg for the other forms.

Each composition described above may further contain other active components unless formulation with the antibody described above causes any adverse interaction.

[6] Pharmaceutical comprising the protein or DNA of the present invention

The protein of the present invention or the DNA encoding the protein of the present invention (hereinafter sometimes simply referred to as the DNA of the present invention) is useful also as an agent for the prevention/treatment of diseases, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), or the like. Where the protein of the present invention is used as the agent for the prevention/treatment described above, it can be prepared into pharmaceutical preparations by publicly known methods. When the DNA of the present invention is used as the agent for the prevention/treatment described above, the DNA of the present invention alone is administered directly, or the DNA is inserted into an appropriate vector such as retrovirus vector, adenovirus vector, adenovirus-associated virus vector, etc., followed by treating in a conventional manner. The DNA of the present invention may be administered as it stands, or by gene gun or through a catheter such as a catheter with a hydrogel, together with aids to assist its uptake.

The protein of the present invention or the DNA of the present invention can be used orally, for example, in the form of tablets which may be sugar coated if necessary, capsules, elixirs, microcapsules, etc., or parenterally in the form of injectable preparations such as a sterile solution, a suspension, etc. in water or with other pharmaceutically acceptable liquid. These preparations can be prepared by mixing the protein of the present invention or the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

The dose of the protein of the present invention may vary depending upon subject to be administered, target disease, conditions, route of administration, etc. Generally speaking, in oral administration, the dose is about 0.1 mg to 100 mg, preferably about 1.0 to 50 mg and more preferably about 1.0 to 20 mg per day in the patient (as 60 kg). In parenteral administration, the single dose may vary depending on subject to be administered, target disease, conditions, route of administration, etc. In the form of, e.g., injectable preparations, it is advantageous to administer intravenously to the patient (as 60 kg) in a daily dose of about 0.01 to about 30 mg, preferably about 0.1 to about 20 mg and more preferably about 0.1 to about 10 mg. For other animal species, the corresponding dose as converted per 60 kg weight can be administered.

The dose of the DNA of the present invention may vary depending upon subject to be administered, target disease, conditions, route of administration, etc. In oral administration, generally speaking, the dose is about 0.1 mg to 100 mg, preferably about 1.0 to 50 mg and more preferably about 1.0 to 20 mg per day in the patient (as 60 kg). In parenteral administration, the single dose may vary depending on subject to be administered, target disease, conditions, route of administration, etc. In the form of, e.g., injectable preparations, it is advantageous to administer intravenously to the patient (as 60 kg) in a daily dose of about 0.01 to about 30 mg, preferably about 0.1 to about 20 mg and more preferably about 0.1 to about 10 mg. For other animal species, the corresponding dose as converted per 60 kg weight can be administered.

[7] Preparation of animal bearing the DNA of the present invention

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:

(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be created by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57Bl/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case oftransfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (1) promoters for DNA derived from viruses (e.g., simian virus, cytomegalovirus, Moloney leukemia virus, JC virus, breast cancer virus, poliovirus, etc.), and promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

The non-human mammal having the exogenous normal DNA of the present invention can be passaged under normal breeding conditions as the DNA-bearing animal by confirming stable retention of the exogenous DNA via crossing.

Transfection of the exogenous DNA of the present invention at the fertilized egg cell stage ensures that the exogenous DNA of the present invention will be present in excess in all germinal and somatic cells of the target mammal. The presence of an excess of the exogenous DNA of the present invention in the animal germ cells after the DNA transfection means that all of the animal progeny will retain an excess of the exogenous DNA of the present invention in all of their germinal and somatic cells. The offspring of animals of this species that inherit the exogenous DNA of the present invention will have an excess of the exogenous DNA of the present invention in all their germinal and somatic cells.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgemic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening of an agent for the treatment of diseases associated with the protein of the present invention.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

A mammal bearing the abnormal exogenous DNA of the present invention is also expected to serve for screening a candidate drug for the treatment of the function inactive type inadaptability of the protein of the present invention, since a free form of the protein of the present invention is increased in such an animal.

Other potential applications of two kinds of the DNA transgenic animals of the present invention described above further include:

(1) Use as a cell source for tissue culture;

(2) Elucidation of the relation to a protein that is specifically expressed or activated by the protein of the present invention, by direct analysis of DNA or RNA in tissues of the DNA transgenic animal of the present invention or by analysis of the polypeptide tissues expressed by the DNA;

(3) Research on the function of cells derived from tissues that are usually cultured only with difficulty, using cells in tissues bearing the DNA cultured by a standard tissue culture technique;
(4) Screening a drug that enhances the functions of cells using the cells described in (3) above; and,
(5) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.
It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.
To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

[8] Knockout animal

The present invention provides a non-human mammal embryonic stem cell bearing the DNA of the present invention inactivated and a non-human mammal deficient in expressing the DNA of the present invention.
Thus, the present invention provides:

(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli*);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (5), wherein the DNA is inactivated by inserting a reporter gene (e.g., β-galactosidase derived from *Escherichia coli*) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).
As the non-human mammal, the same examples as described above apply.
Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.
Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1 % trypsin/1 mM EDTA) at the time of passage to obtain separate single cells, which are then seeded on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector.. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

[8a] Method of screening a compound having a therapeutic/preventive effect on diseases caused by deficiency, damages, etc. of the DNA of the present invention

The non-human mammal deficient in expression of the DNA of the present invention can be employed for screening of a compound having a therapeutic/preventive effect on diseases caused by deficiency, damages, etc. of the DNA of the present invention.

That is, the present invention provides a method of screening a compound having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as given hereinabove apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration route, nature of the test compound, etc.

For screening of the compound having a preventive/therapeutic effect on, e.g., chronic obstructive pulmonary disease or bronchial asthma, the non-human mammal deficient in expression of the DNA of the present invention is subjected to a tobacco smoke or antigen exposure treatment. A test compound is given to the animal before or after the tobacco smoke or antigen exposure treatment and changes in airway responses of the animal, etc. are measured with passage of time.

The compound obtained using the above screening method is a compound selected from the test compounds described above and exhibits a preventive/therapeutic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered to an adult (as 60 kg body weight), the compound is generally administered to the patient with, e.g., chronic obstructive pulmonary disease in a daily dose of about 0.1 to about 100 mg, preferably about 1.0 to about 50 mg and, more preferably about 1.0 to about 20 mg. In parenteral administration, a single dose of said compound or its salt may vary depending upon target subject, target disease, etc. When the compound or its salt is administered to an adult (as 60 kg body weight) with chronic obstructive pulmonary disease in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg weight can be administered.

[8b] Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of the reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds used for the screening.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., organic acids, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that inhibits the promoter activity to the DNA of the present invention can inhibit expression of the protein of the present invention to inhibit the functions of the protein. Thus, the compound or its salt is useful as pharmaceuticals such as preventive/therapeutic agents for, e.g., pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.), and the like.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that promotes the promoter activity to the DNA of the present invention is orally administered an adult (as 60 kg body weight), the compound is administered to the patient with chronic obstructive pulmonary disease normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of promoting the promoter activity to the DNA of the present invention is administered to an adult (as 60 kg body weight) in the form of injectable preparation, it is advantageous to administer the compound intravenously to the patient with chronic obstructive pulmonary disease in a daily dose of about 0.01 to about 30 mg, preferably about 0.1 to about 20 mg and more preferably about 0.1 to about 10 mg. For other animal species, the corresponding dose as converted per 60 kg weight can be administered.

On the other hand, when the compound that inhibits the promoter activity to the DNA of the present invention is orally administered an adult (as 60 kg body weight), the compound is administered to the patient with chronic obstructive pulmonary disease normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to an adult (as 60 kg body weight) in the form of injectable preparation, it is advantageous to administer the compound intravenously to the patient with chronic obstructive pulmonary disease in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of preventive/therapeutic agent for these diseases.

Also, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein of the present invention itself.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A: adenine
T : thymine
G : guanine
C: cytosine
R : adenine (A) or guanine (G)
Y : cytosine (C) or thymine (T)
K : guanine (G) or thymine (T)
M : adenine (A) or cytosine (C)
S: guanine (G) or cytosine (C)
W : adenine (A) or thymine (T)
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine
pGlu : pyroglutamic acid

Substituents, protecting groups and reagents generally used in this specification are presented as the codes below.

Me : methyl group
Et : ethyl group
Bu : butyl group
Ph : phenyl group

TC : thiazolidine-4(R)-carboxamido group
Tos : p-toluenesulfonyl
CHO : formyl
Bzl : benzyl
$Cl_2$-Bzl : 2,6-dichlorobenzyl
Bom : benzyloxymethyl
Z : benzyloxycarbonyl
Cl-Z : 2-chlorobenzyloxycarbonyl
Br-Z : 2-bromobenzyl oxycarbonyl
Boc : t-butoxycarbonyl
DNP : dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboxyimide
DCC : N,N'-dicyclohexylcarbodiimide

The sequence identification numbers in the sequence listing of the specification indicates the following sequence, respectively.

[SEQ ID NO: 1] This shows the partial amino acid sequence of rat CLCA1 protein.
[SEQ ID NO: 2] This shows a base sequence of DNA encoding the partial amino acid sequence of rat CLCA1 protein having the amino acid sequence represented by SEQ ID NO: 1.
[SEQ ID NO: 3] This shows the base sequence of primer I used in EXAMPLE 1.
[SEQ ID NO: 4] This shows the base sequence of primer 2 used in EXAMPLE 1.
[SEQ ID NO: 5] This shows the base sequence of primer 3 used in EXAMPLE 1 and EXAMPLE 3.
[SEQ ID NO: 6] This shows the base sequence of primer 4 used in EXAMPLE 1.
[SEQ ID NO: 7] This shows the base sequence of primer 5 used in EXAMPLE 1.
[SEQ ID NO: 8] This shows the base sequence of primer 6 used in EXAMPLE 1.
[SEQ ID NO: 9] This shows the base sequence of primer 7 used in EXAMPLE 1.
[SEQ ID NO: 10] This shows the base sequence of primer 8 used in EXAMPLE 2.
[SEQ ID NO: 11] This shows the base sequence of primer 9 used in EXAMPLE 2.
[SEQ ID NO: 12] This shows the base sequence of TaqMan probe used in EXAMPLE 2.
[SEQ ID NO: 13] This shows the base sequence of primer 10 used in EXAMPLE 3.
[SEQ ID NO: 14] This shows the base sequence of primer 11 used in EXAMPLE 3.
[SEQ ID NO: 15] This shows the base sequence of T7 promoter primer used in EXAMPLE 1 and EXAMPLE 3.
[SEQ ID NO: 16] This shows the base sequence of M13RV primer used in EXAMPLE 3.
[SEQ ID NO: 17] This shows the base sequence of primer 12 used in EXAMPLE 3.
[SEQ ID NO: 18] This shows the base sequence of primer 13 used in EXAMPLE 3.
[SEQ ID NO: 19] This shows the base sequence of primer AP1 used in EXAMPLE 3.
[SEQ ID NO: 20] This shows the base sequence of primer AP2 used in EXAMPLE 3.
[SEQ ID NO: 21] This shows the base sequence of primer U19 primer used in EXAMPLE 1.
[SEQ ID NO: 22] This shows the amino acid sequence of rat CLCA1 protein.
[SEQ ID NO: 23] This shows a base sequence of DNA encoding rat CLCA1 protein having the amino acid sequence represented by SEQ ID NO: 22.
[SEQ ID NO: 24] This shows the base sequence of primer 14 used in EXAMPLE 3.
[SEQ ID NO: 25] This shows the base sequence of primer 15 used in EXAMPLE 3.

The transformant, Escherichia coli TOP10/pCR-BluntII-rCLCA1 obtained in EXAMPLE 3 later described has been on deposit since January 9, 2002 under the Accession Number FERM BP-7846 at the National Institute of Advanced Industrial Science and Technology, International Patent Organism Depositary, located at Central 6, 1-1-1 Higashi, Tsukuba, Ibaraki, Japan (postal code 305-8566), and since December 18, 2001 has been on deposit at the Institute for Fermentation (IFO), located at 2-17-85, Juso-honmachi, Yodogawa-ku, Osaka-shi, Osaka, Japan (postal code 532-8686) under the Accession Number IFO 16742.

EXAMPLES

The present invention is described in more detail below with reference to EXAMPLES, but is not deemed to limit the scope of the present invention thereto.

EXAMPLE 1

Cloning of rat CLCA1 gene

Based on the consensus sequences among the base sequence of human CLCA1 gene [Genomics, 54, 200 (1998)], the base sequence of mouse gob-5 gene [Biochem. Biophys. Res. Commun., 255, 347 (1999)] and the base sequence of porcine CLCA1 gene [Physiol. Genomics, 3, 101 (2000)], 5 primers [primer 1 (SEQ ID NO: 3), primer 2 (SEQ ID NO: 4), primer 3 (SEQ ID NO: 5), primer 4 (SEQ ID NO: 6) and primer 5 (SEQ ID NO: 7)] were designed and synthesized. Next, the total RNA was prepared from rat gastric mucosal tissue using ISOGEN (manufactured by Wako Pure Chemical Industries, Ltd.). From 200 ng of the total RNA as a starting material, cDNA was synthesized in 100 μl of a reaction solution using TaqMan Gold RT-PCR Kit (manufactured by Applied Biosystems, Inc.). Using this cDNA as the template, PCR was carried out in combination of primers 1 and 4, primers 1 and 5, primers 2 and 3 and primers 3 and 5. Thereafter, it was confirmed by agarose electrophoresis that the DNA fragment having an expected size was amplified. The reaction was carried out using Takara Ex Taq (manufactured by Takara Shuzo Co., Ltd.) on a Gene Amp PCR System 9700 thermal cycler (manufactured by Perkin-Elmer), first by reacting at 94°C for 1 minute and then by repeating the following cycle 35 times, one cycle to set to include 94°C for 10 seconds, 65°C for 30 seconds, 72°C for 2 minutes and 30 seconds and finally by reacting at 72°C for 10 minutes. The resulting DNA fragment was cloned onto pT7 Blue-T vector (manufactured by Novagen). Further cycle sequencing was performed using T7 promoter primer (SEQ ID NO: 15), U19 primer (SEQ ID NO: 21) and two synthetic primers [primer 6 (SEQ ID NO: 8) and primer 7 (SEQ ID NO: 9)]. The reaction product thus obtained was sequenced for its base sequence on fluorescence DNA Sequencer 377 (manufactured by Perkin-Elmer). As a result, the sequence of 1879 bases (SEQ ID NO: 2) was identified to be the partial base sequence of rat CLCA1 gene. Based on the base sequence represented by SEQ ID NO: 2, a sequence of 625 amino acids was deduced (SEQ ID NO: 1). The partial amino acid sequence had 75% homology to the corresponding sequence of human CLCA1 and 88% homology to the corresponding sequence of mouse gob-5.

EXAMPLE 2

Induction of expression of rat CLCA1 gene in rat primary airway epithelial cells by addition of IL-13

Primary airway epithelial cells were isolated from Wistar rat by a modification of the Kitamura's method (Soshiki Baiyo-no-Gijutsu (Technology of Tissue Culture) [2nd edition], edited by The Japanese Tissue Culture Association, Asakura Printing Co., Ltd.) and plated on a 48-well collagen-coated plate (manufactured by Becton, Dickinson & Co.). A fresh medium was replenished every 2 other days. After the cells became confluent, mouse IL-13, human IL-13, mouse IL-4, human IL-4 and human IL-9 were added thereto in a final concentration of 1, 5 and 25 ng/ml, respectively. Eight days after the addition, the total RNA was extracted using RNeasy 96 Kit (manufactured by QIAGEN). Using the total RNA as the starting material, cDNA was synthesized in 100 μl of a reaction solution by reverse transcription. Using 10 μl of the reaction product, a copy number of the rat CLCA1 genes was determined by TaqMan PCR using ABI PRISM 7700 Sequence Detector (manufactured by Applied Biosystems, Inc.). The primers [primer 8 (SEQ ID NO: 10), primer 9 (SEQ ID NO: 11)] and TaqMan probe (SEQ ID NO: 12) used to detect the gene level were designed using a Primer Express program. As a reporter dye for TaqMan probe, 6-carboxyfluorescein (FAM) was used. A copy number of rat GAPDH genes was measured as an internal standard using TaqMan Rodent GAPDH Control Reagents VIC Probe (manufactured by Applied Biosystems, Inc.). To eliminate nonspecific amplification, a reverse transcriptase-free sample was treated as well. A copy number of the rat CLCA1 genes was calculated per GAPDH gene according to the following equation.

(Copy number of the genes in a reverse transcriptase-containing sample)

- (Copy number of the genes in a reverse transcriptase-free sample)

= (Copy number of the genes)

Thus, it was found that expression of rat CLCA1 was induced in rat primary airway epithelial cells dependently only on IL-13 (FIG. 1).

EXAMPLE 3

(1) Cloning of the full-length rat CLCA1 gene

Using a mRNA purification kit (manufactured by Amersham Pharmacia Biotech), mRNA was prepared from the total RNA of rat gastric mucosal tissue prepared in EXAMPLE 1. Using 1 μg of this mRNA as the starting material, Adaptor-ligated cDNA was synthesized on a Marathon cDNA Amplification Kit (manufactured by Clontech). PCR was performed for 5'-RACE, using this cDNA as the template together with primer 10 (SEQ ID NO: 13) and primer AP1 (SEQ ID NO: 19)(manufactured by,Clontech). Using the reaction solution as the template together with primer 11 (SEQ ID NO: 14) and primer AP2 (SEQ ID NO: 20) (manufactured by Clontech), nested PCR was conducted and the DNA fragment thus amplified was cloned onto pCRII-TOPO vector (manufactured by Invitrogen). Cycle sequencing was carried out using T7 promoter primer (SEQ ID NO: 15) and M13RV primer (SEQ ID NO: 16), and the reaction product obtained was sequenced for its base sequence on fluorescence DNA Sequencer 3100 (manufactured by Applied Biosystems, Inc.). As a result, any base sequence corresponding to the N-terminal amino acid sequence of rat CLCA1 was not found. Thus, primer 12 (SEQ ID NO: 17) was further synthesized and using Adaptor-ligated cDNA as the template together with primer AP2 (manufactured by Clontech), PCR was performed and the DNA fragment thus amplified was directly sequenced for its base sequence on fluorescence DNA Sequencer 3100 (manufactured by Applied Biosystems, Inc.). As a result, a base sequence corresponding to the N-terminal amino acid sequence of rat CLCA1 was found.

Also, PCR was performed for 3'-RACE, using Adaptor-ligated cDNA as the template together with primer 3 and primer AP1 (SEQ ID NO: 19) (manufactured by Clontech). Using he reaction solution as the template together with primer 13 (SEQ ID NO: 18) and primer AP2 (manufactured by Clontech), nested PCR was carried out. Subsequently, the DNA fragment thus amplified was directly sequenced for its base sequence on fluorescence DNA Sequencer 3100 (manufactured by Applied Biosystems, Inc.). As a result, a base sequence corresponding to the C-terminal amino acid sequence of rat CLCA1 was found.

The foregoing results revealed that the full-length gene for rat CLCA1 had the sequence of 2730 bases (SEQ ID NO: 23). The sequence of 910 amino acids encoded by the base sequence represented by SEQ ID NO: 23 is shown in SEQ ID NO: 22.

A protein having the amino acid sequence represented by SEQ ID NO: 22 was named rat CLCA1 protein. The rat CLCA1 protein showed 76% homology to human CLCA1 and 89% homology to mouse gob-5, respectively, on an amino acid level.

In the amino acid sequence represented by SEQ ID NO: 22, the 268-889 sequence coincided with the 4-625 amino acid sequence in the amino acid sequence represented by SEQ ID NO: 1. In the amino acid sequence represented by SEQ ID NO: 1, the 1-3 sequence or Tyr-Gly-Leu is replaced by Asn-Gln-Arg of the 265-267 sequence in the amino acid sequence represented by SEQ ID NO: 22.

In the base sequence represented by SEQ ID NO: 2, the 1-10 sequence is replaced by AAAACCAACG in the base sequence represented by SEQ ID NO: 23. Also, the 17th T, 224th C, 1787th A and 1868th C are represented by C, T, C and G, respectively, in the corresponding base sequence represented by SEQ ID NO: 23.

(2) Based on these results, PCR was carried out using rat gastric mucus-derived cDNA as the template, in combination with primer 14 (SEQ ID NO: 24) and primer 15 (SEQ ID NO: 25), using Pyrobest DNA Polymerase (manufactured by Takara Shuzo Co., Ltd.) to clone the DNA fragment having the base sequence represented by SEQ ID NO: 23 onto pCR-BluntII-TOPO vector (manufactured by Invitrogen, Inc.). This plasmid was transfected to Escherichia coli TOP10 (manufactured by Invitrogen, Inc.), which was named Escherichia coli TOP 10/pCR-BluntII-rCLCA1.

EXAMPLE 4

Screening of the compound inhibiting expression of CLCA1

Rat airway epithelia cell line (SPOC-1) (American Journal of Respiratory Cell and Molecular Biology, 14, 146, 1996) was plated on a 96-well collagen-coated plate (manufactured by Becton, Dickinson & Co.) in 1 x $10^4$ cells/well, followed by incubation for 4 days. On day 4 of the incubation, recombinant mouse IL-13 (manufactured by R & D Co.) and various concentrations (1, 10 and 100 ?M) of wortmannin (manufactured by CALBIOCHEM, Inc.) were added to each well, followed by incubation for further 24 hours. Cells added with no wortmannin were made control.

After the cells were washed with PBS, 50 ?l/well of Lysis Buffer (QuantiGene High Volume Kit, manufactured by Bayer) was added to the cells, which was then allowed to stand at 37°C for 30 minutes for cell lysis. To each well, 100 μl of Probe Reagent (QuantiGene High Volume Kit, manufactured by Bayer) was added. After agitation through

pipetting, 100 μl was taken for sampling and added to bDNA Capture Plate (QuantiGene High Volume Kit, manufactured by Bayer), which was reacted at 53°C for 16 to 24 hours. After the plate was washed twice with Wash Buffer (QuantiGene High Volume Kit, manufactured by Bayer), 100 μl of Amplifer Working Reagent (QuantiGene High Volume Kit, manufactured by Bayer) was added thereto, followed by reacting at 46°C for 2 hours. The plate was again washed twice with Wash Buffer and 100 μl/well of Label Working Reagent (QuantiGene High Volume Kit, manufactured by Bayer) was added thereto, followed by reacting at 46°C for 2 hours. The plate was brought back to room temperature. After the plate was washed twice with Wash Buffer, 100 μl/well of Substrate Working Reagent (QuantiGene High Volume Kit, manufactured by Bayer) was added thereto, followed by reacting at 46°C for an hour. The plate was brought back to room temperature, and the luminescence intensity was measured with a plate reader. Since the luminescence intensity increases in proportion to the expression level of rat CLCA, the mRNA of rat CLCA1 can be quantified by measuring the luminescence intensity. In control cells added with no wortmannin, the expression of rat CLCA1 is induced by IL-13 to increase the luminescence intensity.

The results are shown in FIG. 2.

The results reveal that wortmannin suppresses the expression of rat CLCA1 dose-dependently.

Thus, the compound that suppresses the expression of CLCA1 can be screened using the aforesaid screening.

INDUSTRIAL APPLICABILITY

The protein and polynucleotide of the present invention are useful as diagnostic markers for pulmonary and breast disease associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like. Inhibitors obtained by the screening with the use of the protein or the polynucleotide and neutralizing antibodies inhibiting the activity of the protein can be used as agents for the prevention/treatment of, for example, pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like.

In addition, the antisense polynucleotides of the present invention can suppress expression of the protein of the present invention, and are useful for the preventive and/or therapeutic agent for, e.g., pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like. Further, the various polynucleotides of the present invention are useful as diagnosis and prevention/treatment for pulmonary and breast diseases associated with pulmonary and airway inflammation or respiratory diseases [e.g., chronic obstructive pulmonary disease (e.g., chronic bronchitis, pulmonary emphysema, diffuse panbronchiolitis, intrinsic asthma, etc.), bronchial asthma, cystic fibrosis, hypersensitivity pneumonitis, pulmonary fibrosis, etc.], inflammatory bowel disease, allergic conjunctivitis, rhinitis (e.g., allergic rhinitis, pollinosis, acute rhinitis, chronic rhinitis, hypertrophic rhinitis, atrophic rhinitis, dry anterior rhinitis, vasomotor rhinitis, cancrum nasi, sinusitis, etc.) or the like.

## Claims

1. A protein containing the same or substantially the same amino acid sequence as the amino acid sequence represented by SEQ ID NO: 1, or a salt thereof.

2. A protein consisting of the amino acid sequence represented by SEQ ID NO: 1, or a salt thereof.

3. A protein containing the same or substantially the same amino acid sequence as the amino acid sequence represented by SEQ ID NO: 22, or a salt thereof.

4. A protein consisting of the amino acid sequence represented by SEQ ID NO: 22, or a salt thereof.

5. A partial peptide of the protein according to claim 1 or 3, or a salt thereof.

6. A polynucleotide containing a polynucleotide encoding the protein according to claim 1 or 3, or the partial peptide according to claim 5.

7. The polynucleotide according to claim 6, which is a DNA.

8. A DNA consisting of the base sequence represented by SEQ ID NO: 2.

**9.** A DNA consisting of the base sequence represented by SEQ ID NO: 23.

**10.** A recombinant vector containing the polynucleotide according to claim 7.

**11.** A transformant transformed by the recombinant vector according to claim 10.

**12.** A method of manufacturing the protein according to claim 1 or 3 or the partial peptide according to claim 5, or a salt thereof, which comprises culturing the transformant according to claim 11, producing/accumulating the protein according to claim 1 or 3 or the partial peptide according to claim 5, or a salt thereof, and collecting the same.

**13.** A diagnostic product comprising the polynucleotide according to claim 7.

**14.** An antibody to the protein according to claim 1 or 3 or the partial peptide according to claim 5, or a salt thereof.

**15.** A pharmaceutical comprising the antibody according to claim 14.

**16.** A diagnostic product comprising the antibody according to claim 14.

**17.** An antisense polynucleotide comprising a complementary or substantially complementary base sequence to that of the polynucleotide according to claim 7, or a part thereof.

**18.** A pharmaceutical comprising the antisense polynucleotide according to claim 17.

**19.** A method of screening a compound or its salt that inhibits the activity of the protein according to claim 1 or 3 or the partial peptide according to claim 5, or a salt thereof, which comprises using the protein according to claim 1 or 3 or the partial peptide according to claim 5, or a salt thereof.

**20.** A kit for screening a compound or its salt that inhibits the activity of the protein according to claim 1 or 3 or the partial peptide according to claim 5, or a salt thereof, comprising the protein according to claim 1 or 3 or the partial peptide according to claim 5, or a salt thereof.

**21.** A compound or its salt that inhibits the activity of the protein according to claim 1 or 3 or the partial peptide according to claim 5, or a salt thereof, which is obtainable using the screening method according to claim 19 or the screening kit according to claim 20.

**22.** A pharmaceutical comprising the compound or its salt according to claim 21.

**23.** A method of screening a compound or its salt that inhibits expression of a gene for the protein according to claim 1 or 3, which comprises using the polynucleotide according to claim 6.

**24.** The screening method according to claim 23, wherein a cytokine is further used.

**25.** The screening method according to claim 24, wherein the cytokine is IL-13.

**26.** A kit for screening a compound or its salt that inhibits expression of a gene for the protein according to claim 1 or 3, which is **characterized by** comprising the polynucleotide according to claim 6.

**27.** A method of screening a compound or its salt that inhibits expression of a gene for the protein according to claim 1 or 3, which comprises determining expression levels of a gene for the protein according to claim 1 or 3 or the partial peptide according to claim 5, when (i) a cell capable of producing the protein according to claim I or 3 or the partial peptide according to claim 5 is cultured in the presence of a cytokine and (ii) a mixture of the cell and a test compound is cultured in the presence of a cytokine, and comparing the expression levels.

**28.** A compound or its salt that inhibits expression of a gene for the protein according to claim 1 or 3, which is obtainable using the screening method according to claim 23 or 27 or the screening kit according to claim 26.

**29.** A pharmaceutical comprising the compound or its salt according to claim 28.

**30.** The pharmaceutical according to claim 15, 18,22 or 29, which is an agent for the prevention/treatment of chronic obstructive pulmonary disease or bronchial asthma.

**31.** The diagnostic product according to claim 13 or 16, which is a diagnostic product of chronic obstructive pulmonary disease or bronchial asthma.

**32.** A method of preventing/treating chronic obstructive pulmonary disease or bronchial asthma, which comprises administering an effective dose of the compound or its salt according to claim 21 or 28 to a mammal.

**33.** Use of the compound or its salt according to claim 21 or 28 for manufacturing an agent for the prevention/treatment of chronic obstructive pulmonary disease or bronchial asthma.

**34.** An agent for the prevention/treatment of pulmonary and breast diseases, comprising a compound or its salt that inhibits the activity of the protein according to claim 1 or 3 or the partial peptide according to claim 5, or a salt thereof.

Fig. 1

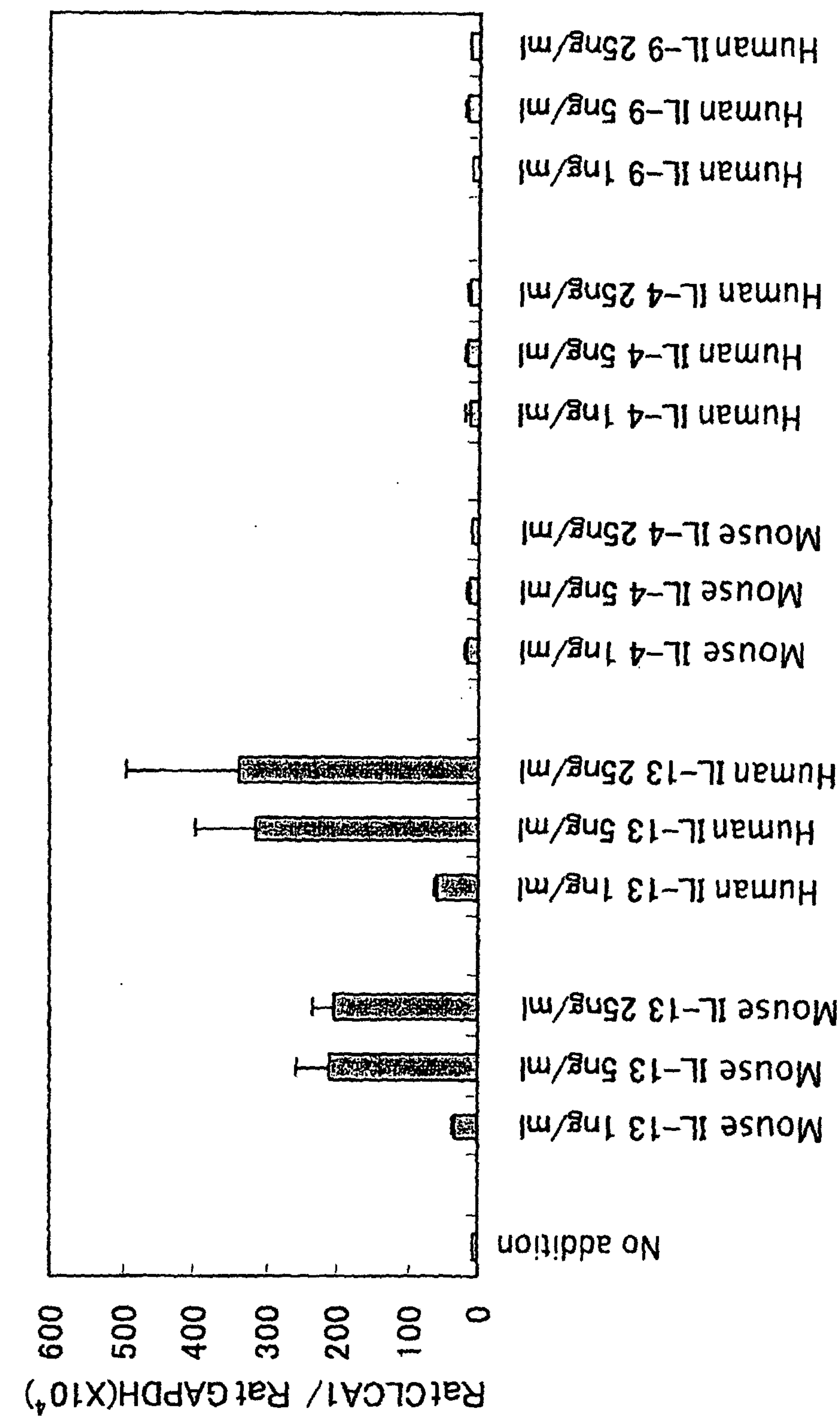
RatCLCA1/ Rat GAPDH(X10$^4$)
600
500
400
300
200
100
0
No addition
Mouse IL-13 1ng/ml
Mouse IL-13 5ng/ml
Mouse IL-13 25ng/ml
Human IL-13 1ng/ml
Human IL-13 5ng/ml
Human IL-13 25ng/ml
Mouse IL-4 1ng/ml
Mouse IL-4 5ng/ml
Mouse IL-4 25ng/ml
Human IL-4 1ng/ml
Human IL-4 5ng/ml
Human IL-4 25ng/ml
Human IL-9 1ng/ml
Human IL-9 5ng/ml
Human IL-9 25ng/ml

Fig. 2

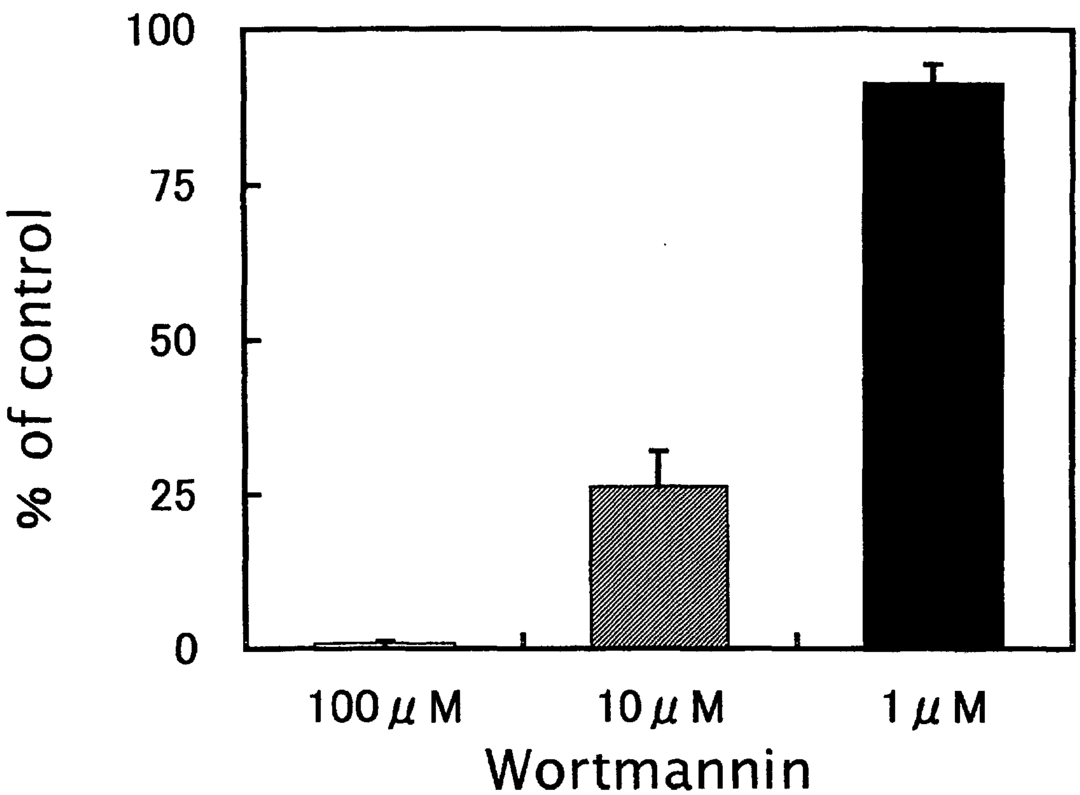

## INTERNATIONAL SEARCH REPORT

International application No.
PCT/JP02/11417

A. CLASSIFICATION OF SUBJECT MATTER
Int.Cl[7] C07K14/47, C12N15/12, C12N5/10, C12N1/15, C12N1/19, C12N1/21, C12P21/02, C07K16/18, C12Q1/02, A61K38/16, A61K39/395, A61K45/00, A61P11/00

According to International Patent Classification (IPC) or to both national classification and IPC

B. FIELDS SEARCHED

Minimum documentation searched (classification system followed by classification symbols)
Int.Cl[7] C07K14/47, C12N15/12, C12N5/10, C12N1/15, C12N1/19, C12N1/21, C12P21/02, C07K16/18, C12Q1/02, A61K38/16, A61K39/395, A61K45/00, A61P11/00

Documentation searched other than minimum documentation to the extent that such documents are included in the fields searched

Electronic data base consulted during the international search (name of data base and, where practicable, search terms used)
SwissProt/PIR/GeneSeq, Genbank/EMBL/DDBJ/GeneSeq, WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| X | Gruber A.D. et al., Genomic cloning, molecular characterization, and functional analysis of human CLCA1, the first human member of the Family of $Ca^{2+}$-activated $Cl^-$channel proteins, Genomics, 1998, Vol.54, No.2, pages 200 to 214 | 1-20,23-31, 33-34 |
| X | KOMIYA T., Cloning and identification of the gene gob-5, which is expressed in intestinal goblet cells in mice, Biochem.Biophys.Res.Commun., 1999, Vol.255, No.2, pages 347 to 351 | 1-20,23-31, 33-34 |
| X | WO 01/38530 A1 (Takeda Chemical Industries, Ltd.), 31 May, 2001 (31.05.01), Seq. 1-4 & JP 2002-010791 A & EP 1234878 A1 | 1-20,23-31, 33-34 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 January, 2003 (30.01.03) | 12 February, 2003 (12.02.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No. PCT/JP02/11417

C (Continuation). DOCUMENTS CONSIDERED TO BE RELEVANT

| Category* | Citation of document, with indication, where appropriate, of the relevant passages | Relevant to claim No. |
|---|---|---|
| X | Gaspar K.J. et al., Cloning a chloride conductance mediator from the apical membrane of porcine ileal enterocytes, Physiol. Genomics., 2000, Vol.3, No.2, pages 101 to 111 | 1-20,23-31, 33-34 |
| X | US 6309857 B1 (Cornell Research Foundation Inc.), 30 October, 2001 (30.10.01), Seq. 27-28, 33-34 (Family: none) | 1-20,23-31, 33-34 |
| X<br>Y | Pahan K. et al., Interleukin-10 and interleukin-13 inhibit proinflammatory cytokine-induced ceramide production through the activation of phosphatidylinositol 3-kinase, J.Neurochem., 2000, Vol.75, No.2, pages 576 to 582 | 28-29<br>30,33 |
| X<br>Y | Wright K. et al., Cytokine-induced apoptosis in epithelial HT-29 cells is independent of nitric oxide formation, J.Biol.Chem., 1999, Vol.274, No.24, pages 17193 to 17201 | 28-29<br>30,33 |

Form PCT/ISA/210 (continuation of second sheet) (July 1998)

**INTERNATIONAL SEARCH REPORT**

International application No.
PCT/JP02/11417

**Box I Observations where certain claims were found unsearchable (Continuation of item 2 of first sheet)**

This international search report has not been established in respect of certain claims under Article 17(2)(a) for the following reasons:

1. [X] Claims Nos.: 32
   because they relate to subject matter not required to be searched by this Authority, namely:
   It involves methods for treatment of the human body by therapy.

2. [X] Claims Nos.: 21-22
   because they relate to parts of the international application that do not comply with the prescribed requirements to such an extent that no meaningful international search can be carried out, specifically:
   (See extra sheet.)

3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.

[ ] No protest accompanied the payment of additional search fees.

Form PCT/ISA/210 (continuation of first sheet (1)) (July 1998)

**INTERNATIONAL SEARCH REPORT**

International application No.
PCT/JP02/11417

Continuation of Box No.I-2 of continuation of first sheet(1)

The compound as set forth in claim 21 is specified as "a compound or its salt inhibiting the activity of a protein as set forth in claim 1 or 3 or the peptide fragment as set forth in claim 5 or salts thereof which can be obtained by using the screening method as set forth in claim 19 or the screening kit as set forth in claim 20" and thus involves any compounds obtained by the screening method.

However, no specific compound obtained by the screening method is presented in the description. Therefore, claim 21 is neither supported by the description nor disclosed therein. Although the common technical knowledge at the point of the application is considered, it is completely unknown what specific compounds are involved therein and what are not. Thus, claim 21 is described in an extremely unclear manner.

Such being the case, no meaningful search can be made on the invention as set forth in claim 21.

For the same reason, no meaningful search can be made on the invention as set forth in claim 22 which depends on claim 21.

Form PCT/ISA/210 (extra sheet) (July 1998)

SEQUENCE LISTING

<110> Takeda Chemical Industries, Ltd.

<120> Novel Protein, DNA and Use Thereof

<130> P02-139

<150> JP 2001-337864
<151> 2001-11-02

<150> JP 2001-380099
<151> 2001-12-13

<150> JP 2002-10035
<151> 2002-01-18

<160> 25

<210> 1
<211> 625
<212> PRT
<213> Rat

<400> 1
```
Tyr Gly Leu Cys Asn Leu Arg Ser Thr Trp Glu Val Ile Gln Glu Ser
                5                   10                  15
Glu Asp Phe Lys Gln Thr Thr Pro Met Thr Ala Gln Pro Pro Ala Pro
            20                  25                  30
Thr Phe Ser Leu Leu Gln Thr Arg Gln Arg Ile Val Cys Leu Val Leu
        35                  40                  45
Asp Lys Ser Gly Ser Met Gln Ser Asp Asn Arg Leu Asn Arg Met Asn
    50                  55                  60
Gln Ala Ser Arg Leu Phe Leu Leu Gln Thr Val Glu Gln Gly Ser Trp
65                  70                  75                  80
Val Gly Met Val Thr Phe Asp Ser Thr Ala Tyr Val Gln Ser Glu Leu
                85                  90                  95
Thr Gln Leu Asn Ser Gly Ala Asp Arg Asp Leu Leu Ile Lys Arg Leu
            100                 105                 110
Pro Thr Val Ala Ser Gly Gly Thr Ser Ile Cys Ser Gly Leu Gln Ala
        115                 120                 125
Ala Phe Thr Ser Ile Lys Lys Lys Tyr Pro Thr Asp Gly Ala Glu Ile
    130                 135                 140
Val Leu Leu Thr Asp Gly Glu Asp Asn Thr Ile Ser Ser Cys Phe Asp
145                 150                 155                 160
Leu Val Lys Asn Ser Gly Ala Ile Ile His Thr Val Ala Leu Gly Pro
                165                 170                 175
Ser Ala Ala Lys Glu Leu Glu Gln Leu Ser Lys Met Thr Gly Gly Leu
            180                 185                 190
Gln Thr Tyr Ser Ser Asp Gln Ile Gln Asn Asn Gly Leu Val Asp Ala
        195                 200                 205
Phe Ala Ala Leu Ser Ser Gly Asn Ala Ala Ile Ser Gln His Ser Ile
    210                 215                 220
Gln Leu Glu Ser Arg Gly Val Asn Leu Gln Asn Lys Gln Trp Met Asn
225                 230                 235                 240
Gly Ser Val Ile Val Asp Ser Thr Val Gly Lys Asp Thr Leu Phe Leu
                245                 250                 255
Val Thr Trp Thr Thr Asn Ser Pro Ser Ile Phe Ile Trp Asp Pro Ser
            260                 265                 270
Gly Val Gln Gln Ser Gly Phe Val Leu Asp Thr Asn Thr Lys Val Ala
        275                 280                 285
Tyr Leu Gln Val Pro Gly Ile Ala Lys Val Gly Phe Trp Lys Tyr Ser
    290                 295                 300
Ile Gln Ala Ser Ser Gln Thr Leu Thr Leu Thr Val Thr Ser Arg Ala
```

```
305                 310                 315                 320
Ala Ser Ala Thr Leu Pro Pro Ile Thr Val Thr Pro Val Val Asn Lys
                325                 330                 335
Asn Thr Gly Lys Phe Pro Ser Pro Val Thr Val Tyr Ala Ser Ile Arg
            340                 345                 350
Gln Gly Ala Ser Pro Ile Leu Arg Ala Ser Val Thr Ala Leu Ile Glu
        355                 360                 365
Ser Val Asn Gly Lys Thr Val Thr Leu Glu Leu Leu Asp Asn Gly Ala
    370                 375                 380
Gly Ala Asp Ala Thr Lys Asn Asp Gly Val Tyr Ser Arg Phe Phe Thr
385                 390                 395                 400
Ala Phe Asp Ala Asn Gly Arg Tyr Ser Ile Lys Ile Trp Ala Leu Gly
                405                 410                 415
Gly Val Thr Ala Asp Arg Gln Arg Met Ala Pro Gln Arg Asn Gly Val
            420                 425                 430
Met Tyr Ile Asp Gly Trp Ile Glu Asp Asp Gly Glu Ile Lys Met Asn
        435                 440                 445
Pro Pro His Pro Glu Thr Gly Asn Val Gln Asp Ser Gln Val Cys Phe
    450                 455                 460
Ser Arg Thr Ser Ser Gly Gly Ser Phe Val Ala Thr Asn Val Pro Ala
465                 470                 475                 480
Ala Pro Ile Pro Asp Leu Phe Pro Pro Cys Gln Ile Thr Asp Leu Lys
                485                 490                 495
Ala Ser Ile Gln Gly Gln Asn Leu Val Asn Leu Thr Trp Thr Ala Pro
            500                 505                 510
Gly Asp Asp Tyr Asp His Gly Arg Ala Ser Ser Tyr Ile Ile Arg Ile
        515                 520                 525
Ser Thr Ser Ile Val Asp Leu Arg Asn Asn Phe Ser Thr Ser Leu Glu
    530                 535                 540
Val Asn Thr Thr Asp Leu Ile Pro Lys Glu Ala Ser Ser Glu Glu Met
545                 550                 555                 560
Phe Glu Phe Glu Leu Asp Asn Ser Phe Gly Asn Gly Thr Asp Val Phe
                565                 570                 575
Ile Ala Ile Gln Ala Val Asp Lys Ser Asn Leu Lys Ser Glu Ile Ser
            580                 585                 590
Asn Ile Ala Arg Val Ser Leu Phe Ile Pro Ala Gln Glu Pro Pro Glu
        595                 600                 605
Asp Ser Thr Pro Ser Tyr Pro Glu Val Ser Ile Asn Ser Thr Ile Pro
    610                 615                 620
Gly
625

<210> 2
<211> 1879
<212> DNA
<213> Rat

<400> 2
catatgggtt atgcaatctc cgaagcactt gggaagtcat ccaggaatct gaggacttca     60
agcaaaccac tcccatgaca gcccagccac ccgcacccac cttctcactg ctgcaaacca    120
gacaaagaat cgtgtgcctc gttcttgata agtccgggag catgcagagc gataaccgtc    180
ttaaccgaat gaatcaggca agccggcttt tcctgctgca gaccgtggag cagggatcct    240
gggtcgggat ggtgaccttc gacagtaccg cctatgtaca aagcgaactc acacagttaa    300
acagtggtgc tgacagagac ctgctgatca agcgcttacc cacagtagct tcaggaggga    360
cgtctatatg ctctgggctt caggcagcat ttacatcgat aaagaagaag tacccgacgg    420
atggagctga aatcgtgctg ctgaccgacg gggaagataa caccattagc agctgctttg    480
acctggtgaa gaacagtggg gccatcattc acacagtggc cctgggaccg tctgccgcta    540
aagagcttga acagctgtcc aaaatgacag gaggcttgca aacgtattct tccgatcaga    600
ttcagaacaa cggtcttgtt gatgctttcg cagctctctc ctcaggaaat gcagctatct    660
ctcagcactc catccagctg gagagcagag gagttaatct ccagaataag cagtggatga    720
atggctcagt gatcgtggac agcacagtgg ggaaggacac cttgtttctt gtcacctgga    780
ctacgaattc tccttcaata tttatctggg atcccagtgg agtgcaacaa agtggttttg    840
tactagacac caacaccaag gtggcctacc tccaagtccc aggcatagcc aaggttggct    900
tttggaaata cagcattcaa gcgagctcac agactctcac tttgactgtc acctcccggg    960
```

```
cagcaagcgc tacactgcct ccaattacag tgaccccagt agtgaataag aacacaggga  1020
aattccccag ccctgtaacc gtgtatgcaa gcattcgcca aggagcctcg cctattctca  1080
gggccagtgt cacagccctg attgaatctg tgaatggaaa aacagtaacc ctggaattac  1140
tggataacgg agcaggtgcc gatgctacca agaatgatgg tgtctactca aggtttttta  1200
cagcttttga tgcaaatggt agatacagca tcaaaatatg ggctctggga ggagttactg  1260
cagacagaca gagaatggcc cctcagagga atggagtcat gtacatagat ggctggattg  1320
aagacgacgg tgaaataaaa atgaatccac cacaccctga aactggtaat gttcaagaca  1380
gccaagtgtg cttcagcagg acatcctcag gggatcgtt tgtggccacc aacgtccccg  1440
cagctcccat tcctgacctc tttccacctt gccaaatcac tgacctgaag gctagcattc  1500
aagggcagaa ccttgtgaat ctgacgtgga cggctcctgg ggatgattac gaccacggga  1560
gagcttccag ttacatcatc cgaataagca cgagtatcgt cgatctcaga aacaacttca  1620
gcacctcact cgaagtgaac actaccgatc tcatccccaa ggaggccagc tccgaggaaa  1680
tgtttgagtt tgaactggac aacagctttg gaaatggcac agatgtcttc attgctatcc  1740
aggctgtgga taagtccaac ctgaaatcag aaatctccaa cattgcacgg gtgtctctgt  1800
tcatccccgc tcaggagccg ccagaagact caactccttc ttatcccgaa gtcagcatca  1860
acagcaccat tcctggcat                                                1879
```

<210> 3
<211> 18
<212> DNA
<213> Artificial Sequence

<220>
<223> Primer

<400> 3
atgcaatctc cgaagcac 18

<210> 4
<211> 22
<212> DNA
<213> Artificial Sequence

<220>
<223> Primer

<400> 4
atgccaggaa tggtgctgtt ga 22

<210> 5
<211> 20
<212> DNA
<213> Artificial Sequence

<220>
<223> Primer

<400> 5
tggacrgctc ctggggatga 20

<210> 6
<211> 20
<212> DNA
<213> Artificial Sequence

<220>
<223> Primer

<400> 6
tcatccccag gagcygtcca 20

<210> 7
<211> 22
<212> DNA

<213> Artificial Sequence

<220>
<223> Primer

<400> 7
gtgcaatgtt ggakatttct ga 22

<210> 8
<211> 22
<212> DNA
<213> Artificial Sequence

<220>
<223> Primer

<400> 8
tggccttcgg acagcattta ca 22

<210> 9
<211> 27
<212> DNA
<213> Artificial Sequence

<220>
<223> Primer

<400> 9
cctgagaata ggcgaggctc cttggcg 27

<210> 10
<211> 22
<212> DNA
<213> Artificial Sequence

<220>
<223> Primer

<400> 10
tcaagcgagc tcacagactc tc 22

<210> 11
<211> 20
<212> DNA
<213> Artificial Sequence

<220>
<223> Primer

<400> 11
aattggaggc agtgtagcgc 20

<210> 12
<211> 23
<212> DNA
<213> Artificial Sequence

<220>
<223> Probe

<400> 12
tttgactgtc acctcccggg cag 23

<210> 13

<211> 22
<212> DNA
<213> Artificial Sequence

<220>
<223> Primer

<400> 13
tgtcatggga gtggtttgct tg 22

<210> 14
<211> 18
<212> DNA
<213> Artificial Sequence

<220>
<223> Primer

<400> 14
gtgcttcgga gattgcat 18

<210> 15
<211> 19
<212> DNA
<213> Artificial Sequence

<220>
<223> Primer

<400> 15
aatacgactc actataggg 19

<210> 16
<211> 17
<212> DNA
<213> Artificial Sequence

<220>
<223> Primer

<400> 16
caggaaacag ctatgac 17

<210> 17
<211> 27
<212> DNA
<213> Artificial Sequence

<220>
<223> Primer

<400> 17
cgttggatga gggcttcatc ttctggc 27

<210> 18
<211> 20
<212> DNA
<213> Artificial Sequence

<220>
<223> Primer

<400> 18
aggctgtgga taagtccaac 20

<210> 19
<211> 27
<212> DNA
<213> Artificial Sequence

<220>
<223> Primer

<400> 19
ccatcctaat acgactcact atagggc 27

<210> 20
<211> 23
<212> DNA
<213> Artificial Sequence

<220>
<223> Primer

<400> 20
actcactata gggctcgagc ggc 23

<210> 21
<211> 19
<212> DNA
<213> Artificial Sequence

<220>
<223> Primer

<400> 21
gttttcccag tcacgacgt 19

<210> 22
<211> 910
<212> PRT
<213> Rat

<400> 22
```
Met Gly Ser Leu Lys Ser Pro Val Phe Leu Leu Val Leu Tyr Leu Leu
                5                   10                  15
Glu Gly Val Leu Ser Asn Ser Leu Ile Gln Leu Asn Asn Asn Gly Tyr
            20                  25                  30
Glu Gly Ile Val Ile Ala Ile Asp His Asp Val Pro Glu Asp Glu Ala
        35                  40                  45
Leu Ile Gln Arg Ile Lys Asp Met Val Thr Gln Ala Ser Pro Tyr Leu
    50                  55                  60
Phe Glu Ala Thr Gly Lys Arg Phe Tyr Phe Lys Asn Val Ala Ile Leu
65                  70                  75                  80
Ile Pro Glu Asn Trp Asn Thr Lys Pro Glu Tyr Lys Arg Pro Lys Leu
                85                  90                  95
Glu Thr Leu Lys Asn Ala Asp Val Leu Val Ser Thr Met Ser Pro Ile
            100                 105                 110
Gly Asn Asp Glu Pro Tyr Thr Glu His Ile Gly Ala Cys Gly Glu Arg
        115                 120                 125
Gly Ile Arg Ile His Leu Thr Pro Asp Phe Leu Ala Gly Lys Lys Gln
    130                 135                 140
Thr Glu Tyr Gly Pro Gln Asp Arg Thr Phe Val His Glu Trp Ala His
145                 150                 155                 160
Phe Arg Trp Gly Val Phe Asp Glu Tyr Asn Asn Asn Glu Lys Phe Tyr
                165                 170                 175
Leu Ser Asn Gly Lys Pro Gln Ala Val Arg Cys Ser Ala Thr Ile Thr
            180                 185                 190
```

```
Gly Lys His Val Val Arg Arg Cys Gln Gly Gly Ser Cys Val Thr Asn
        195                 200                 205
Gly Lys Cys Val Ile Asp Arg Val Thr Gly Leu Tyr Lys Asp Asn Cys
    210                 215                 220
Val Phe Ile Pro Asp Lys Asn Gln Arg Glu Lys Ala Ser Ile Met Phe
225                 230                 235                 240
Asn Gln Asn Ile Asn Ser Val Val Glu Phe Cys Thr Glu Lys Asn His
                245                 250                 255
Asn Lys Glu Ala Pro Asn Ala Gln Asn Gln Arg Cys Asn Leu Arg Ser
            260                 265                 270
Thr Trp Glu Val Ile Gln Glu Ser Glu Asp Phe Lys Gln Thr Thr Pro
        275                 280                 285
Met Thr Ala Gln Pro Pro Ala Pro Thr Phe Ser Leu Leu Gln Thr Arg
    290                 295                 300
Gln Arg Ile Val Cys Leu Val Leu Asp Lys Ser Gly Ser Met Gln Ser
305                 310                 315                 320
Asp Asn Arg Leu Asn Arg Met Asn Gln Ala Ser Arg Leu Phe Leu Leu
                325                 330                 335
Gln Thr Val Glu Gln Gly Ser Trp Val Gly Met Val Thr Phe Asp Ser
            340                 345                 350
Thr Ala Tyr Val Gln Ser Glu Leu Thr Gln Leu Asn Ser Gly Ala Asp
        355                 360                 365
Arg Asp Leu Leu Ile Lys Arg Leu Pro Thr Val Ala Ser Gly Gly Thr
    370                 375                 380
Ser Ile Cys Ser Gly Leu Gln Ala Ala Phe Thr Ser Ile Lys Lys Lys
385                 390                 395                 400
Tyr Pro Thr Asp Gly Ala Glu Ile Val Leu Leu Thr Asp Gly Glu Asp
                405                 410                 415
Asn Thr Ile Ser Ser Cys Phe Asp Leu Val Lys Asn Ser Gly Ala Ile
            420                 425                 430
Ile His Thr Val Ala Leu Gly Pro Ser Ala Ala Lys Glu Leu Glu Gln
        435                 440                 445
Leu Ser Lys Met Thr Gly Gly Leu Gln Thr Tyr Ser Ser Asp Gln Ile
    450                 455                 460
Gln Asn Asn Gly Leu Val Asp Ala Phe Ala Ala Leu Ser Ser Gly Asn
465                 470                 475                 480
Ala Ala Ile Ser Gln His Ser Ile Gln Leu Glu Ser Arg Gly Val Asn
                485                 490                 495
Leu Gln Asn Lys Gln Trp Met Asn Gly Ser Val Ile Val Asp Ser Thr
            500                 505                 510
Val Gly Lys Asp Thr Leu Phe Leu Val Thr Trp Thr Thr Asn Ser Pro
        515                 520                 525
Ser Ile Phe Ile Trp Asp Pro Ser Gly Val Gln Gln Ser Gly Phe Val
    530                 535                 540
Leu Asp Thr Asn Thr Lys Val Ala Tyr Leu Gln Val Pro Gly Ile Ala
545                 550                 555                 560
Lys Val Gly Phe Trp Lys Tyr Ser Ile Gln Ala Ser Ser Gln Thr Leu
                565                 570                 575
Thr Leu Thr Val Thr Ser Arg Ala Ala Ser Ala Thr Leu Pro Pro Ile
            580                 585                 590
Thr Val Thr Pro Val Val Asn Lys Asn Thr Gly Lys Phe Pro Ser Pro
        595                 600                 605
Val Thr Val Tyr Ala Ser Ile Arg Gln Gly Ala Ser Pro Ile Leu Arg
    610                 615                 620
Ala Ser Val Thr Ala Leu Ile Glu Ser Val Asn Gly Lys Thr Val Thr
625                 630                 635                 640
Leu Glu Leu Leu Asp Asn Gly Ala Gly Ala Asp Ala Thr Lys Asn Asp
                645                 650                 655
Gly Val Tyr Ser Arg Phe Phe Thr Ala Phe Asp Ala Asn Gly Arg Tyr
            660                 665                 670
Ser Ile Lys Ile Trp Ala Leu Gly Gly Val Thr Ala Asp Arg Gln Arg
        675                 680                 685
Met Ala Pro Gln Arg Asn Gly Val Met Tyr Ile Asp Gly Trp Ile Glu
    690                 695                 700
```

Asp Asp Gly Glu Ile Lys Met Asn Pro Pro His Pro Glu Thr Gly Asn
705 710 715 720
Val Gln Asp Ser Gln Val Cys Phe Ser Arg Thr Ser Ser Gly Gly Ser
725 730 735
Phe Val Ala Thr Asn Val Pro Ala Ala Pro Ile Pro Asp Leu Phe Pro
740 745 750
Pro Cys Gln Ile Thr Asp Leu Lys Ala Ser Ile Gln Gly Gln Asn Leu
755 760 765
Val Asn Leu Thr Trp Thr Ala Pro Gly Asp Asp Tyr Asp His Gly Arg
770 775 780
Ala Ser Ser Tyr Ile Ile Arg Ile Ser Thr Ser Ile Val Asp Leu Arg
785 790 795 800
Asn Asn Phe Ser Thr Ser Leu Glu Val Asn Thr Thr Asp Leu Ile Pro
805 810 815
Lys Glu Ala Ser Ser Glu Glu Met Phe Glu Phe Glu Leu Asp Asn Ser
820 825 830
Phe Gly Asn Gly Thr Asp Val Phe Ile Ala Ile Gln Ala Val Asp Lys
835 840 845
Ser Asn Leu Lys Ser Glu Ile Ser Asn Ile Ala Arg Val Ser Leu Phe
850 855 860
Ile Pro Ala Gln Glu Pro Pro Glu Asp Ser Thr Pro Ser Tyr Pro Glu
865 870 875 880
Val Ser Ile Asn Ser Thr Ile Pro Gly Ile His Val Leu Asn Ile Val
885 890 895
Trp Lys Trp Leu Gly Glu Met His Val Thr Leu Gly Leu His
900 905 910

<210> 23
<211> 2730
<212> DNA
<213> Rat

<400> 23
atgggatctt tgaagagtcc tgtcttcctc ttggtcctct accttctgga aggggttctg 60
agcaattccc tcatccagtt gaacaacaat ggctatgaag gcatcgtcat tgccatagac 120
cacgatgtgc cagaagatga agccctcatc caacggataa aggacatggt gactcaggcc 180
tctccatacc tgtttgaagc tacagggaaa agattttact tcaaaaatgt tgccattttg 240
attcctgaga attggaacac aaaacctgaa tataagaggc caaaacttga aaccttaaaa 300
aatgctgatg tccttgtgtc aacaatgagc cccataggta atgatgagcc ctacaccgag 360
cacataggag catgtggaga aagggggatc aggattcacc tgactcctga cttcttagca 420
ggaaagaagc agactgagta tggcccacaa gacaggacgt ttgttcatga gtgggctcac 480
ttccgatggg ggtgtttga cgagtacaac aacaacgaga agttctactt gtccaacgga 540
aagccccagg cagtaagatg ttcggcaacc attaccggta aacatgtagt tcgtcggtgc 600
cagggaggca gttgcgtcac taacggaaag tgcgtaatcg acagagtaac gggactgtat 660
aaagataact gtgtattcat accagataaa aaccagagag agaaggcgtc catcatgttt 720
aaccaaaata tcaactctgt ggttgaattc tgtacagaaa aaaatcacaa taaagaagcc 780
cccaatgccc aaaaccaacg atgcaacctc cgaagcactt gggaagtcat ccaggaatct 840
gaggacttca agcaaaccac tcccatgaca gcccagccac ccgcacccac cttctcactg 900
ctgcaaacca gacaaagaat cgtgtgcctc gttcttgata agtccgggag catgcagagc 960
gataaccgtc ttaaccgaat gaatcaggca agccggcttt tcctgctgca gactgtggag 1020
cagggatcct gggtcgggat ggtgaccttc gacagtaccg cctatgtaca aagcgaactc 1080
acacagttaa acagtggtgc tgacagagac ctgctgatca agcgcttacc cacagtagct 1140
tcaggaggga cgtctatatg ctctgggctt caggcagcat ttacatcgat aaagaagaag 1200
tacccgacgg atggagctga aatcgtgctg ctgaccgacg gggaagataa caccattagc 1260
agctgctttg acctggtgaa gaacagtggg gccatcattc acacagtggc cctgggaccg 1320
tctgccgcta aagagcttga acagctgtcc aaaatgacag gaggcttgca aacgtattct 1380
tccgatcaga ttcagaacaa cggtcttgtt gatgctttcg cagctctctc ctcaggaaat 1440
gcagctatct ctcagcactc catccagctg gagagcagag gagttaatct ccagaataag 1500
cagtggatga atggctcagt gatcgtggac agcacagtgg ggaaggacac cttgtttctt 1560
gtcacctgga ctacgaattc tccttcaata tttatctggg atcccagtgg agtgcaacaa 1620
agtggttttg tactagacac caacaccaag gtggcctacc tccaagtccc aggcatagcc 1680
aaggttggct tttggaaata cagcattcaa gcgagctcac agactctcac tttgactgtc 1740
acctcccggg cagcaagcgc tacactgcct ccaattacag tgaccccagt agtgaataag 1800
aacacaggga aattccccag ccctgtaacc gtgtatgcaa gcattcgcca aggagcctcg 1860

```
cctattctca gggccagtgt cacagccctg attgaatctg tgaatggaaa aacagtaacc  1920
ctggaattac tggataacgg agcaggtgcc gatgctacca agaatgatgg tgtctactca  1980
aggtttttta cagcttttga tgcaaatggt agatacagca tcaaaatatg ggctctggga  2040
ggagttactg cagacagaca gagaatggcc cctcagagga atggagtcat gtacatagat  2100
ggctggattg aagacgacgg tgaaataaaa atgaatccac cacaccctga aactggtaat  2160
gttcaagaca gccaagtgtg cttcagcagg acatcctcag gggatcgtt tgtggccacc  2220
aacgtccccg cagctcccat tcctgacctc tttccacctt gccaaatcac tgacctgaag  2280
gctagcattc aagggcagaa ccttgtgaat ctgacgtgga cggctcctgg ggatgattac  2340
gaccacggga gagcttccag ttacatcatc cgaataagca cgagtatcgt cgatctcaga  2400
aacaacttca gcacctcact cgaagtgaac actaccgatc tcatccccaa ggaggccagc  2460
tccgaggaaa tgtttgagtt tgaactggac aacagctttg gaaatggcac agatgtcttc  2520
attgctatcc aggctgtgga taagtccaac ctgaaatcag aaatctccaa cattgcccgg  2580
gtgtctctgt tcatccccgc tcaggagccg ccagaagact caactccttc ttatcccgaa  2640
gtcagcatca acagcacgat tcctggcatc cacgtgctga atatagtgtg gaagtggcta  2700
ggggaaatgc atgtgacact aggtttgcac                                  2730

<210> 24
<211> 25
<212> DNA
<213> Artificial Sequence

<220>
<223> Primer

<400> 24
gggaaagctg aagatgggat ctttg         25

<210> 25
<211> 27
<212> DNA
<213> Artificial Sequence

<220>
<223> Primer

<400> 25
ttttacagct tcacgtgtta ctacatc        27
```